# EUROPEAN PATENT SPECIFICATION

(11) **EP 2 076 244 B1**
(45) Date of publication and mention of the grant of the patent: **07.12.2016**
(21) Application number: 07873522.2
(22) Date of filing: 10.10.2007
(51) Int. Cl.: A61K 9/127, A61K 9/19, A61K 9/00, A61K 47/48, A61K 9/51

(54) **AQUEOUS SYSTEMS FOR THE PREPARATION OF LIPID-BASED PHARMACEUTICAL COMPOUNDS; COMPOSITIONS, METHODS, AND USES THEREOF**
WÄSSRIGE SYSTEME FÜR DIE ZUBEREITUNG PHARMAZEUTISCHER VERBINDUNGEN AUF FETTBASIS, ZUSAMMENSETZUNGEN, VERFAHREN UND IHRE VERWENDUNG
SYSTÈMES AQUEUX POUR LA PRÉPARATION DE COMPOSÉS PHARMACEUTIQUES À BASE LIPIDIQUE ET LEURS COMPOSITIONS, PROCÉDÉS ET UTILISATIONS

(30) Priority: 10.10.2006 US 850446 P; 21.08.2007 US 957022 P
(43) Date of publication of application: 08.07.2009
(73) Proprietor: Jina Pharmaceuticals Inc., Libertyville IL 60048 (US)
(72) Inventor: ALI, Shoukath, M., Lake Bluff, IL 60048 (US); AHMAD, Moghis, U., Wadsworth, IL 60083 (US); AHMAD, Ateeq, Wadsworth, IL 60083 (US); SHEIKH, Saifuddin, Waukegan, IL 60085 (US); AHMAD, Imran, Wadsworth, IL 60083 (US)
(74) Representative: Glawe, Delfs, Moll
(86) International application number: PCT/US2007/080984
(87) International publication number: WO 2008/127358

(56) References cited:
- EP-A1- 0 560 138
- WO-A1-02/32400
- WO-A1-2005/044229
- WO-A2-02/058622
- US-A- 5 616 341
- US-A1- 2005 214 378
- US-A1- 2006 110 441
- DATABASE WPI Week 198226, Derwent Publications Ltd., London, GB; AN 1982-53655E & JP S5 782311 A (TANABE SEIYAKU CO) 22 May 1982
- R. M. Pashley ET AL: "De-Gassed Water Is a Better Cleaning Agent", The Journal of Physical Chemistry B, vol. 109, no. 3, 1 January 2005 (2005-01-01), pages 1231-1238, XP055213096, ISSN: 1520-6106, DOI: 10.1021/jp045975a

## Description

### FIELD OF THE INVENTION

The invention relates to compositions comprising active components or compounds, e.g., pharmaceutical compounds, and lipids, including, e.g., complexes, micelles, emulsions, liposomes or lipidic particle, and mixture of micelles and vesicles. The invention further relates to their methods of preparation. By way of example, in some embodiments, the invention relates to compositions comprising amphotericin B, with or without deoxycholate, and one or more lipids, their methods of preparation in an aqueous system, and their uses for the treatment of diseases, such as mammalian diseases. In some embodiments, the invention relates to compositions comprising, e.g., immunosuppressants such as tacrolimus, anticancer compounds such as docetaxel or paclitaxel, or any other compound of the taxane family, and one or more lipids, their methods of preparation in the absence of organic solvents, and their uses for treatment, e.g., of mammalian diseases. Methods according to the present invention are suitable for practice on an industrial manufacturing scale, and may be practiced, e.g. , as a continuous process. Other significant advantages of these methods include simplicity, speed of particle formation, ease of scaling to large volumes, the formation of lipid suspensions of high concentration and defined particle size and the ability to aqueous systems in the encapsulation of pharmaceutically active compounds having poor water solubility.

### BACKGROUND OF THE INVENTION

Most lipidic preparation systems involve the use of organic solvents such as dimethylsulfoxide, dimethylformamide, methylene chloride, chloroform, ethanol or methanol. Organic solvents can pose health risks, e.g., for production workers, and removal of organic solvents is generally a cumbersome process. Hence there is a need for processes for preparation of lipid based formulations without the need for from organic solvents.

Polyene antibiotics provide one example a class of active pharmaceutical compounds having limited solubility in aqueous systems. Polyene antibiotics are widely used in the treatment of both pre-systemic and systemic fungal infections. They are produced by several different species of Streptomyces. Recent interest in these antibiotics is stimulated due to their synergistic antifungal action with other agents (Medoff, G. and Kobayashi, G.S. 1975) and by reports of antitumor action (Valeriote, F. et al. 1976). In particular, polyene antibiotics such as amphotericin B (AmB) and Nystatin (Nys) have remained the most effective and widely used agents in the treatment of fungal infections. In addition several, but not all, of these agents have been shown to have immunoadjuvant properties (Hammarstron, L. and Smith, C.I.E., 1977; Little, J.R. et al. 1978).

The polyene antibiotics target sterols, specifically ergosterol, which is the abundant and main sterol of fungal membranes. The different types of polyene antibiotics display different modes of action, despite that they share a common target. The larger polyenes like amphotericin and nystatin form together with ergosterol pore structures in the plasma membrane which collapse vital ion gradients, thereby killing the cells. The smaller uncharged filipin also destroys the membrane barrier, but by a completely different mechanism. Filipin forms large complexes with sterols between the leaflets of the lipid bilayer, resulting in breakage of the membrane (De Kruijff and Demel, 1974). Natamycin like the other polyene-antibiotics specifically binds to ergosterol in the membrane, but this does not result in a loss of barrier function.

Amphotericin B is a parental antifungal antibiotic produced as a fermentation by-product of streptomyces nodusus, a soil actinomycete. It binds to sterols in the cell membranes of both fungal and mammalian cell. It is usually fungistatic *in vivo* but can have fungicidal activity at high concentrations or against extremely susceptible organisms. Its higher affinity for ergosterol, the sterol found in fungal cell membranes, over cholesterol, the sterol found in human cell membranes, allows amphotericin B to be used systematically. As a result of this binding, fungal membrane integrity is impaired, causing the loss of intracellular potassium and other cellular contents. Some adverse reactions to amphotericin B, such as electrolyte loss and nephrotoxicity, are an extension of its pharmacologic action, while anaphylactoid infusion-related reactions may be related to stimulation and release of prostaglandin synthesis. Anemia may be secondary to an inhibition of erythropoietin production.

Amphotericin B is widely used for severe life-threatening fungal infections. Its use limited by a dose-dependent nephrotoxicity, manifested by a reduction in glomerular filtration rate and tubular dysfunction. An elevated excretion of creatinine associated with amphotericin B is not only a marker for renal dysfunction but is also linked to a substantial risk for the use of hemodialysis and a higher mortality rate; therefore, amphotericin B nephrotoxicity is not benign complication and its prevention is essential. (Deray, G. et al. Nephrologie, 2002).

Amphotericin B is poorly soluble in water, alcohols, chloroform, and other common halocarbon solvents. While amphotericin B is an effective fungicide, it is dangerously toxic at concentrations slightly above the therapeutic concentration. Encapsulation in liposomes appears to reduce the in vivo toxicity to mammalian cells, and leaving the fungicidal activity relatively unaltered (F.C.Szoka et.al., 1987). Liposomes have been used to encapsulate a large variety of compounds which exhibits poor solubility or exhibits unacceptable toxicity at therapeutic dosages. The effects of liposome encapsulation on cytotoxicity and fungicidal activity of compounds such as amphotericin B are dependent on the particular liposome structure (e.g., SUV, MLV etc.) and their method of preparation.

Development of new formulations using new lipid compositions is needed to improve the efficacy and to reduce the toxicity associated with compositions such as polyene antibiotics, and particularly with amphotericin B, with or without deoxycholate.

Taxanes are a unique class of hydrophobic anticancer agents that exhibit cytotoxic activity by binding to tubulin and promoting inappropriately stable, non-functional microtubule formation (Schiff PB et. al. 1979). Interference with microtubule function leads to disrupted mitosis and cell death. Certain taxanes, e.g., paclitaxel and docetaxel, are approved for human use for the treatment of breast cancer, ovarian cancer, non-small cell lung cancer and prostrate cancer. The dose limiting toxicity profiles for these agents are somewhat different; paclitaxel has been most widely associated with peripheral neuropathies and myalgias/ athralgias, where as docetaxel most commonly results in fluid retention that may be dose-limiting in some cases (Hennenfent, K.L et al 2006).

The taxanes, including but not limited to paclitaxel and docetaxel, are practically insoluble in water and require a complex solvent system for commercial formulation. Cremophor EL, a polyoxyethylated castor oil vehicle, and dehydrated ethanol USP (1 :1, v/v) are used as solvents in the commercial formulation of paclitaxel, while polysorbate 80 (Tween 80 detergent) is employed in the formulation of docetaxel. Although these solvents systems are biologically and pharmacologically acceptable, they have known to have side effects, including acute hypersensitivity reactions and peripheral neuropathies. In addition, several reports have linked these solvents to alterations in the pharmacokinetic profiles of both paclitaxel and docetaxel (ten Tije, AJ et al. 2003).

Several formulations have been made to solublize the taxanes and to circumvent the toxicities associated with it. All of these formulations, including lipid-based formulations (for example, liposomes), have required use of organic solvents to solubilize the active compound during the formulation process (Straubinger, et.al. US5,415,868, 1995; Bisery, et al US6, 146,663, 2000). As noted above, the use of organic solvents results in a cumbersome process and hence an organic solvent-free formulation is needed to overcome the problems associated with the existing formulations.

### SUMMARY OF THE INVENTION

The subject-matter for which protection is sought is set out in the appended claims. The embodiments and examples of the description which do not fall within the scope of said claims are provided for illustrational purposes only. The present invention relates to new methods of preparing active compounds complexed with lipids. The complex interaction may be ionic or lipophilic. In all the embodiments of the present invention, the complex formation takes place in aqueous media. In some embodiments, the present invention comprises a composition comprising a complex comprising at least one active agent, such as a polyene antibiotic, an immunosuppressant agent such as tacrolimus or a taxane or taxane derivative and one or more lipids.

An object of the present invention is to provide lipid formulations or complexes comprising at least one active component and at least one lipid, e.g., a phospholipid, formed without using organic solvent.

The amount of phospholipid included in a lipid complex according to the present invention is not limited to any particular amount or percentage (e.g., by weight) of the final composition or complex. In some embodiments, the proportion of the at least one phospholipid is between about 5% to about 98% of a final lipid complex (e.g., a commercially usable form) by weight. In some preferred embodiments, the amount of the at least one phospholipid is between 10% to 90% of the lipid complex by weight.

In certain embodiments, a lipid formulation system according to the present invention has a pH of between about 4.0 and 8.0. In some preferred embodiments, the pH is between about 4.5 and 7.5.

A lipid formulation of the present invention is not limited to any particular use or application. For example, a lipid formulation of an active component according to the present description comprising a pharmaceutically active ingredient can be used for different pharmaceutical applications. An aqueous system of the present invention can also be used in the formation of unloaded lipid complexes (e.g., without any encapsulated active ingredient), for use, e.g., as controls for complexes comprising active components.

In some embodiments, the present invention comprises a composition comprising a complex comprising at least one anticancer agent and one or more lipids. Examples of anticancer agents include but are not limited to docetaxel, paclitaxel, epirubicin, endoxifen and the like.

As for example, it is possible to encapsulate or entrap tacrolimus, in the inventive liposome system, such a pharmaceutical product is used, e.g. , as an immunosuppressant or for the treatment of skin infection. Such a pharmaceutical product is particularly suitable for injection or oral usage. Furthermore, the known active ingredients are for the treatment of cancer, liver disease, kidney diseases, AIDS, bacterial, fungal and viral infections.

In some embodiments, the present invention comprises a composition comprising a complex comprising at least one immunosuppressant agent and one or more lipids. Examples of immunosuppressant include but not limited to tacrolimus and sacrolimus.

In some embodiments, the polyene antibiotic of a composition according to the present invention is amphotericin B with or without deoxycholate, while in some preferred embodiments; the amphotericin B deoxycholate is Fungizone^{®} antibiotic. In some embodiments the amphotericin B deoxycholate is prepared from amphotericin B and sodium deoxycholate.

In some embodiments, the one or more lipids of a composition according to the present invention comprise one or more of cholesterol, cholesteryl sulfate and its salts (e.g. , sodium salt), cholesteryl hemisuccinate.

In some embodiments, the one or more lipids of a composition according to the present invention comprises one or more of fatty acids having a chain length of about C₄-C34. In some embodiments, one or more fatty acid chains are unsaturated, while in some embodiments, one or more of the fatty acid chains are saturated. In some embodiments, one or more of the fatty acids are in salt form, while in some embodiments; one or more of the fatty acids are in acidic form. In some embodiments, one or more fatty acids are in the form of an ester.

In some embodiments, one or more lipids of a composition according to the present invention comprise a phospholipid. In some preferred embodiments, one or more of the lipids of the composition comprises a phosphatidylcholine or phosphatidylglycerol, while in some preferred embodiments; one or more of the lipids of the composition comprises a phosphatidylethanolamine, phosphatidylserine, phosphatdylinositol, or phosphatidic acid. In some preferred embodiments, one or more lipids of the present invention comprise a soybean phospholipid. In some particularly preferred embodiments, a soybean phospholipid used in the methods and compositions of the present invention comprises a large concentration of phosphatidylcholine. In still more particularly preferred embodiments, a soybean phospholipid used in the methods and compositions of the present invention contains at least 90% by weight phosphatidylcholine. In some embodiments, one or more phospholipids are pegylated (PEG) derivatives of phospholipids. In certain embodiments, one or more of the lipids of the composition comprise a pegylated derivative of a distearoylphosphatidylglycerol, a dimyristoylphosphatidylglycerol, or a dioleoylphosphatidylglycerol phospholipid.

In some embodiments, a composition according to the present invention further comprises polyethylene glycol (PEG). In some embodiments, the PEG has an average molecular weight ranging from 200-20,000, while in certain preferred embodiments, the average molecular weight of the PEG is in the range of 500-2000.

In some embodiments, a composition according to the present invention comprises active compound (for example amphotericin B, with or without sodium deoxycholate), cholesterol or cholesterol derivatives and one or more phospholipids. In certain preferred embodiments, the composition comprises sodium deoxycholate, and the mole ratio of active compound (for example, amphotericin B) to sodium deoxycholate is about 1:2. In some embodiments in which the composition comprises a cholesterol derivative, the cholesterol derivative is cholesteryl sulfate. In some embodiments wherein the phospholipid comprises soy phosphatidylcholine or hydrogenated phosphatidylcholine. In some preferred embodiments, the mole ratio of active compound (for example, amphotericin B) and cholesterol or cholesterol derivative is in the range of about 1:1 and 1:10, while in certain particularly preferred embodiments, the mole ratio of active compound (for example, amphotericin B) and cholesterol or cholesterol derivative is in between about 1:1 and 1:5.

In some embodiments, one or more lipids of a composition according to the present invention comprise hydrogenated soy phosphatidylcholine, wherein the mole ratio of active compound (for example, amphotericin B) and hydrogenated soy phosphatidylcholine is in between about 1:5 and 1:80. In certain preferred embodiments, the mole ratio of active compound (for example, amphotericin B) and hydrogenated soy phosphatidylcholine is in between about 1:5 and 1:60.

In some embodiments, a composition according to the present invention comprises active compound (for example, amphotericin B, with or without sodium deoxycholate) at a concentration of from about 0.5mg/mL to about 25mg/mL while in some preferred embodiments, the active compound (for example, amphotericin B with or without deoxycholate) of the composition is at a concentration of from about 1 mg/mL to about 10 mg/mL. In some particularly preferred embodiments, the composition of the invention comprises active compound (for example, amphotericin B, with or without deoxycholate) is at a concentration of about 1 mg/mL to about 5 mg/mL.

In some embodiments, a composition according to the present invention comprises a total lipid concentration or proportion of from about 2.5% by weight to about 95% by weight, while in some preferred embodiments; the composition comprises a total lipid concentration of from about 5% by weight to about 95% by weight. In certain particularly preferred embodiments, the composition comprises a total lipid concentration of from about 10% by weight to about 90% by weight.

In some embodiments, a composition according to the present invention comprises active compound (for example, amphotericin B), and total lipids including sodium deoxycholate (if used) having molar ratio ranging from about 1:10 to about 1:100, while in some embodiments, the molar ratio is in between about 1: 20 to about 1:70.

In some embodiments, a composition according to the present invention comprises active compound (for example, amphotericin B) and total lipid(s) including sodium deoxycholate having a weight-to-weight ratio ranging from about 1:1 to about 1:100, while in certain preferred embodiments, the ratio is in between about 1:10 to about 1:60.

In some embodiments, a composition according to the present invention comprises a complex selected from the group consisting of a micelle and an emulsion. In certain preferred embodiments, the composition comprises a plurality of micelles, wherein said micelles are in the form of monomeric, dimeric, polymeric or mixed micelles.

In some embodiments, a composition according to the present invention comprises complexes, liposomes, micelles, and/or vesicles that have a diameter of about 20 microns or less, while in some embodiments, the complexes, liposomes, micelles, and/or vesicles that have a diameter of about 10 microns or less. In some embodiments, the complexes, liposomes, micelles, and/or vesicles have a diameter of about 5 microns or less, while in some embodiments, the complexes, liposomes, micelles, and/or vesicles have a diameter of about 1 micron or less. In some embodiments, the complexes, liposomes, micelles, and/or vesicles have a diameter of about 500 nm or less, while in some embodiments, the complexes, liposomes, micelles, and/or vesicles have a diameter of about 200 nm or less. In some preferred embodiments, the complexes, liposomes, micelles, and/or vesicles have a diameter of about 100 nm or less.

The present description is not limited to any particular form of composition comprising the complex of the description. For example, in some embodiments, a complex in a composition according to the present invention is in a lyophilized form. In some embodiments, the composition further comprises a cryoprotectant. In certain preferred embodiments, the cryoprotectant comprises one or more sugars, while in particularly preferred embodiments; the one or more sugars comprise trehalose, maltose, lactose, sucrose, glucose, and/or dextran.

In some embodiments, a complex in a composition according to the present description is in a powder form, while in some embodiments, the complex is in a solution form. In some embodiments, the complex is in a suspension form, while in other embodiments, the complex is in an emulsion form, while in still other embodiments, the complex is in a micelle form or mixed micellar form or in a liposome form. In some embodiments, the complex is in a lyophilized or gel form, while in some embodiments, the complex is in a paste form. In some embodiments, the complex is a mixture of mixed micelles, liposomes or vesicles form.

In some embodiments, a composition according to the present description is encapsulated in a capsule. In some preferred embodiments, the capsule is a gel capsule, while in some particularly preferred embodiments; the capsule comprises an enteric coating.

In some embodiments, a complex in a composition according to the present invention is comprises a water insoluble, or poorly water soluble, drug that is not a polyene antibiotic.

In some embodiments, a composition according to the present invention comprises an active component comprising a macro lide, e.g., Tacrolimus ( Knoll, G. A. et al. 1999; Dumont FJ In: Liebermann R, Mukherjee A, eds. 1996). or Sirolimus (Ingle GR, et al. 2000;; Podder H, et al.. 2001). Macro-lides such as Tacrolimus are currently used clinically for the prophylaxis of liver and kidney transplant rejection. In some embodiments, a lipid composition according to the present invention comprises a macrolide and finds use, e.g., in immunosuppression and/or the suppression of transplant rejection. Similarly, in some embodiments, a lipid composition according to the present invention comprises an anticancer drug as an active component, and finds use, e.g., in treatment of cancer diseases.

The methods, compositions and systems of the present invention are not limited to use with or comprising any particular active components or agents. For example, drugs, active agents or therapeutic agents that find use in the methods, compositions and systems of the present invention include, e.g., , agents that act on the peripheral nerves, adrenergic receptors, cholinergic receptors, the skeletal muscles, the cardiovascular system, smooth muscles, the blood circulatory system, synaptic sites, neuroeffector functional sites, endocrine and hormone systems, the immunological system, the reproductive system, the skeletal system, the alimentary and excretory systems, the histamine system and the central nervous system. Suitable active agents may be selected from, for example, proteins, enzymes, and hormones, nucleotides (including sense and antisense oligonucleotides) (e.g., U.S. Patent 6,126,965, 2000), polynucleotide, nucleoproteins, polysaccharides, glycoproteins, lipoproteins, polypeptides, steroids. Active agents can be analgesics, anesthetics, anti-arrhythmic agents, antibiotics, antiallergic agents, antifungal agents, anticancer agents, anticoagulants, antidepressants, antidiabetic agents, anti-epilepsy agents, anti-inflammatory corticosteroids, agents for treating Alzheimer's or Parkinson's disease, antiulcer agents, anti-protozoal agents, anxiolytics, thyroids, anti-thyroids, antiviral, anorectics, bisphosphonates, cardiac inotropic agents, cardiovascular agents, corticosteroids, diuretics, dopaminergic agents, gastrointestinal agents, hemostatics, hyper cholesterol agents, antihypertensive agents (e.g., dihydropyridines), antidepressants, and cox-2 inhibitors, immunosuppressive agents, anti-gout agents, anti-malarials, steroids, terpinoids, triterpines, retinoid, anti-ulcer H2-receptor antagonists, hypoglycemic agents, moisturizers, cosmetics, anti-migraine agents, antimuscarinic agents, anti-inflammatory agents, such as agents for treating rheumatology, arthritis, psoriasis, inflammatory bowel disease, Crohn's disease, or agents for treating demyelinating diseases including multiple sclerosis, ophthalmic agents, vaccines (e.g., against pneumonia, hepatitis A, hepatitis B, hepatitis C, cholera toxin B subunit, influenza virus, typhoid, plasmodium falciparum, diphtheria, tetanus, HSV, tuberculosis, HIV, SARS virus, perpetual pertussis, measeles, mumps and rubella vaccine (MMV), bacterial toxins, vaccinea virus, adenovirus, canary, polio virus, bacillus calmette guerin (BCG), klebsiella pneumonia, etc.), histamine receptor antagonists, hypnotics, kidney protective agents, lipid regulating agents, muscle relaxants, neuroleptics, neurotropic agents, opioid agonists and antagonists, parasympathomimetics, protease inhibitors, prostaglandins, sedatives, sex hormones (e.g., estrogen, androgen), stimulants, sympathomimetics, vasodilators and xanthenes and synthetic analogs of these species. The therapeutic agents can be nephrotoxic, such as cyclosporine and amphotericin B, or cardiotoxic, such as amphotericin B and paclitaxel. Exemplary anticancer agents include melphalan, chlormethine, extramustinephosphate, uramustine, ifosfamide, mannomustine, trifosfamide, streptozotocin, mitobronitol, mitoxantrone (see., e.g., international patent application WO 02/32400), methotrexate, fluorouracil, cytarabine, tegafur, idoxide, taxanes [(e.g., taxol, paclitaxel, etc., see international patent application WO 00/01366; U.S.Patent 5,415,869)], daunomycin or daunorubicin, epirubicin, bleomycin, etoposide, tamoxifen, hydroxytamoxifen, endoxifen carboplatin, cisplatin, paclitaxel, docetaxel, BCNU, vinca alkaloids (e.g., vincristine, vinorelbine (e.g., international patent application WO 03/018018, and the like) camptothecin and derivatives thereof (see, e.g., international patent publication WO 02/058622), SN 38 , irinotecan (see, e.g., international patent publication WO 03/030864, and the like), cytokines, ribozymes, interferons, oligonucleotides and functional anthracyclines, antibodies, cytoxines, doxorubicin, etopside, derivatives of the foregoing. Additional examples of drugs that find use in the methods, compositions and systems of the present invention include, azidothymidine (AZT), acyclovir, tacrolimus, prochlorperzine edisylate, ferrous sulfate, aminocaproic acid, mecamylamine hydrochloride, procainamide hydrochloride, amphetamine sulfate, methamphetamine hydrochloride, benzamphetamine hydrochloride, isoproterenol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erythrityl tetra nitrate, digoxin, isoflurophate, acetazolamide, methazolamide, bendroflumethiazide, chloropromaide, tolazamide, chlormadinone acetate, phenaglycodol, allopurinol, aluminum aspirin, methotrexate, acetyl sulfisoxazole, erythromycin, hydrocortisone, hydrocorticosterone acetate, cortisone acetate, dexamethasone and its derivatives such as betamethasone, triamcinolone, methyl testosterone, 17-β-estradiol, ethinyl estradiol, ethinyl estradiol 3-methyl ether, prednisolone, 17-α-hydroxyprogesterone acetate, 19-norprogesterone, norgestrel, norethindrone, norethisterone, norethiederone, progesterone, norgesterone, norethynodrel, aspirin, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, chlorpromazine, methyldopa, dihydroxyphenylalanine, theophylline, calcium gluconate, ketoprofen, ibuprofen, cephalexin, erythromycin, haloperidol, zomepirac, ferrous lactate, vincamine, diazepam, phenoxybenzamine, diltiazem, milrinone, mandol, quanbenz, hydrochlorothiazide, ranitidine, flurbiprofen, fenufen, fluprofen, tolmetin, alclofenac, mefenamic, flufenamic, difuinal, nimodipine, nitrendipine, nisoldipine, nicardipine, felodipine, lidoflazine, tiapamil, gallopamil, amlodipine, mioflazine, lisinolpril, enalapril, enalaprilat captopril, ramipril, famotidine, nizatidine, sucralfate, etintidine, tetratolol, minoxidil, chlordiazepoxide, diazepam, amitriptyline, and imipramine. Further examples are proteins and peptides which include, but are not limited to, bone morphogenic proteins, insulin, colchicine, glucagon, thyroid stimulating hormone, parathyroid and pituitary hormones, digestive hormones, calcitonin, rennin, prolactin, corticotrophin, thyrotropic hormone, follicle stimulating hormone, chorionic gonadotropin, gonadotropin releasing hormone, bovine somatotropin, porcine somatotropin, oxytocin, vasopressin, GRF, somatostatin, lypressin, pancreozymin, luteinizing hormone, LHRH, LHRH agonists and antagonists, leuprolide, interferon's (e.g., consensus interferon, interferon α-2α, interferon α-2β, α-, β-, or γ- interferon's), interleukins, growth hormones such as human growth hormone and its derivatives such as methione-human growth hormone and desphenylalanine human growth hormone, bovine growth hormone and porcine growth hormone, fertility inhibitors such as the prostaglandins, fertility promoters, growth factors such as insulin-like growth factor, coagulation factors, pancreas hormone releasing factor, analogues and derivatives of these compounds, and pharmaceutically acceptable salts of these compounds, or their analogues or derivatives. The therapeutic agent can be a mixture of drugs or agents (e.g., two or more agents) that can be beneficially co- administered in the liposome formulation.

The inventive method is simple, rapid and less expensive method to produce organic solvent-free aqueous liposome systems, which allow a particularly simple and rapid inspection of foreign particles. Furthermore, the liposome system produced according to the inventive method shows highly reproducible particle sizes, with average particle size below 5 micron, preferably between 50nm and 1 micron. It is also possible to filter the product through sterile filtration known in the art. The duration of the extrusion, or the high pressure split homogenization is chosen to be sufficiently long for the liposomes to show the desired average diameter. Said extrusion, high pressure split homogenization is performed until liposomes possess a mean diameter between 50nm and 1 micron.

The liposome system produced according to the present inventive method can be filled directly in corresponding ampoules in a condition ready to use, and lyophilize the product after the adding the desired amount of carbohydrate known in the art, whereby lyophilization constitute the best method of water drying. This gives liposome system in powder form, which can be re-constituted into the vesicles by the addition of suitable amount of water for injection, normal saline or 5% dextrose with gentle shaking. It is not necessary to subject the liposome system formed after the addition of injectable water to extensive agitation or high pressure split homogenization.

The methods and compositions of the present invention can be used to treat a disease caused by fungal or bacterial infection. In some embodiments, the methods and compositions of the present invention are used to treat a fungal disease caused by at least one of the fungus selected from the group of fungus consisting of Acremonium sp.,Aspergillus fumigatus, Aspergillus pneumonia, Blastomyces dermatitidis, Candida albicans, Candida guillermondi, Candida tropicalis, Coccidioides immitis, Cryptococcus neoformans, Fusarium sp., Histoplasma capsulatum, Mucor mucedo, Rhodotorula sp., Sporothrix schenckii, Acanthamoeba polyphaga, Entomophthora sp., Histoplasma capsulatumm Leishmania brasiliensis, Rhizopus sp., Rhodotorula sp., Torulopsis glabrata, Paracoccidioides brasiliensis. Additional fungal pathogens include Trichosporon, Muco, Alternaria, Bipolaris, Curvularia, etc.

The methods and compositions of the present invention can be used to treat disease caused by a species of Leishmania, for example, in some embodiments, the methods and compositions of the present invention are used to treat Visceral Leishmaniasis.

In some embodiments, the methods and compositions of the present invention can be used to treat a viral infection, e.g. a viral infection caused by human immunodeficiency virus (HIV), herpes simplex viruses (HSV-I and HSV2), hepatitis C virus (HCV) or cytomegalovirus (CMV).

In some embodiments, the present inventions comprise a method of treating a cell with amphotericin B with or without deoxycholate, preparing a composition according as described herein, and exposing the cells to the composition. In some preferred embodiments, the exposing of the cell occurs in vivo, e.g., in a patient or subject.

It is contemplated that in some embodiments, the exposing of a cell in a subject comprises oral delivery of the composition to the subject, while in other embodiments; the exposing of a cell comprises intravenous delivery of the composition to the subject. Routes of delivery of the composition to the subject that find use in the present invention include but are not limited to subcutaneous delivery, parenteral delivery, intraperitoneal delivery, rectal delivery, vaginal delivery and/or topical delivery. In some preferred embodiments, the subject is a mammal. In some particularly preferred embodiments, the mammal is human.

### DEFINITIONS

The term "lipid composition" as used herein refers to amphoteric compounds which are capable of liposome formation, vesicle formation, micelle formation, emulsion formation, and are substantially non-toxic when administered. The lipid composition may include without limitation egg phosphatidylcholine (EPC),egg phosphatidylglycerol (EPG), soy phosphatidylcholine (SPC), hydrogenated soy phosphatidylcholine (HSPC), dimyristoylphosphatidylcholine (DMPC), dimyristoylphosphatidylglycerol (DMPG), Dipalmitoylphosohatidylcholine (DPPC), disteroylphosphatidylglycerol (DSPG), dipalmitoylphosphatidylglycerol (DMPG), cholesterol (Chol), cholesterol sulfate and its salts (CS), cholesterol hemisuccinate and its salts (Chems), cholesterol phosphate and its salts (CP), cholesterylphospholine and other hydroxycholesterol or amino cholesterol derivatives.

As used herein, the term "aqueous" as used in reference to a solvent, fluid, or system, refers to a water-based solvent, fluid or system that does not contain any organic solvents.

As used herein, the term "aqueous system" as used in reference to production of a complex comprising at least one active compound and at least one lipid refers to a process or method of production, or to the set of materials used in such production, that contain or comprise use of water-based solvents and lipids but do not contain or comprise use of organic solvents.

As used herein, the term "organic solvent" refers to a carbon-containing chemical, generally in liquid form, used to dissolve another substance. Examples of organic solvents include but are not limited to alcohols, glycols, ethers, dimethoxyethane, acetone, chloroform, dimethyl sulfoxide, hexane, toluene, tetrahydrofuron (THF), methylene chloride and the like.

The term "encapsulating amount" refers to the amount of lipid necessary to encapsulate the poorly soluble compound and form liposome or lipidic particles of appropriate mean particle size less than 5,000nm in diameter, preferably between 30-1000nm. The encapsulating amount will depend on the pharmaceutically active compounds and process conditions selected, but in general range in between from 2:1 to about 1:100 compound: lipid ratio; preferably about 1:1 to about 1:50.

The term "lipidic particle" as used herein refers to particles of undefined structure which consist of a suitable lipid and an encapsulated or complexed pharmaceutically active compound. Polyene antibiotics at high antibiotic: lipid ratios typically form lipidic particles rather than liposomes, due to the polyene structure and its interaction with the lipid. Lipidic particles may have a lamellar structure but are not required to exhibit any defined structure.

As used herein, the term "effective amount" refers to the amount of an active composition (*e*.*g*., a pharmaceutical compound or composition provided as a component in a lipid formulation) sufficient to effect beneficial or desired results. An effective amount can be administered in one or more administrations, applications or dosages and is not intended to be limited to a particular formulation or administration route.

As used herein, the terms "active" or "pharmaceutically active" as used in reference to an agent, composition, or compound, refers to an agent that, upon administration or application, causes a beneficial, desired, or expected result. The administration may be in one or more administrations, applications or dosages and is not intended to be limited to a particular formulation or administration route. The term is not limited to any particular level of activity. For example, a lipid formulation of an active agent need not have the same level of activity as a different formulation of an active agent, so long as the active agent in the lipid formulation is sufficiently active that an effective amount of the active agent can be administered by administration of the lipid formulation of the agent.

The terms "agent" and "compound" are used herein interchangeably to refer to any atom, molecule, mixture, or more complex composition having an attributed feature. For example, an "active agent" or "active compound" refers to any atom, molecule, preparation, mixture, *etc*., that, upon administration or application, causes a beneficial, desired, or expected result.

As used herein, the term "administration" refers to the act of giving a drug, prodrug, or other active agent, or therapeutic treatment (*e*.*g*., compositions of the present invention) to a physiological system (*e*.*g*., a subject or *in vivo*, *in vitro*, or *ex vivo* cells, tissues, and organs). Exemplary routes of administration to the human body can be through the eyes (ophthalmic), mouth (oral), skin (transdermal), nose (nasal), lungs (inhalant), rectal, vaginal, oral mucosa (buccal), ear, by injection (*e*.*g*., intravenously, subcutaneously, intratumorally, intraperitoneally, *etc*.) and the like. Administration may be in one or more administrations, applications or dosages, and is not intended to be limited to a particular administration route.

As used herein, the term "co-administration" refers to the administration of at least two agent(s) (*e*.*g*., two separate lipid compositions, containing different active compounds) or therapies to a subject. In some embodiments, the co-administration of two or more agents or therapies is concurrent. In other embodiments, a first agent/therapy is administered prior to a second agent/therapy. Those of skill in the art understand that the formulations and/or routes of administration of the various agents or therapies used may vary. The appropriate dosage for co-administration can be readily determined by one skilled in the art. In some embodiments, when agents or therapies are co-administered, the respective agents or therapies are administered at lower dosages than appropriate for their administration alone. Thus, co-administration is especially desirable in embodiments where the co-administration of the agents or therapies lowers the requisite dosage of a potentially harmful (*e*.*g*., toxic) agent(s).

As used herein, the term "toxic" refers to any detrimental or harmful effects on a subject, a cell, or a tissue as compared to the same cell or tissue prior to the administration of the toxicant.

As used herein, the term "pharmaceutical composition" refers to the combination of an active agent (*e*.*g*., an active pharmaceutical compound) with a carrier, inert or active (*e*.*g*., a phospholipid), making the composition especially suitable for diagnostic or therapeutic use *in vitro*, *in vivo* or *ex vivo.*

The terms "pharmaceutically acceptable" or "pharmacologically acceptable," as used herein, refer to compositions that do not substantially produce adverse reactions, *e*.*g*., toxic, allergic, or immunological reactions, when administered to a subject.

As used herein, the term "topically" refers to application of the compositions of the present invention to the surface of the skin and mucosal cells and tissues (*e*.*g*., alveolar, buccal, lingual, masticatory, or nasal mucosa, and other tissues and cells which line hollow organs or body cavities).

As used herein, the term "pharmaceutically acceptable carrier" refers to any of the standard pharmaceutical carriers including, but not limited to, phosphate buffered saline solution, water, emulsions (*e*.*g*., such as an oil/water or water/oil emulsions), and various types of wetting agents, any and all solvents, dispersion media, coatings, sodium lauryl sulfate, isotonic and absorption delaying agents, disintrigrants (*e*.*g*., potato starch or sodium starch glycolate), and the like.. The compositions also can include stabilizers and preservatives. For examples of carriers, stabilizers, and adjuvants. (See *e*.*g*., Martin, Remington's Pharmaceutical Sciences, 15th Ed., Mack Publ. Co., Easton, Pa. (1975), incorporated herein by reference). Moreover, in certain embodiments, the compositions of the present invention may be formulated for horticultural or agricultural use. Such formulations include dips, sprays, seed dressings, stem injections, sprays, and mists.

As used herein, the term "pharmaceutically acceptable salt" refers to any salt (*e*.*g*., obtained by reaction with an acid or a base) of a compound of the present invention that is physiologically tolerated in the target subject (*e*.*g*., a mammalian subject, and/or *in vivo* or *ex vivo*, cells, tissues, or organs). "Salts" of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, sulfonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Examples of bases include, but are not limited to, alkali metal (e.g., sodium) hydroxides, alkaline earth metal (*e*.*g*., magnesium) hydroxides, ammonia, and compounds of formula NW₄⁺, wherein W is C₁₋₄ alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, chloride, bromide, iodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like. For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

The term "Polyethylene glycol (PEG)" includes polymers of lower alkylene oxide, in particular ethylene oxide (polyethylene glycols) having an esterifiable hydroxyl group at least at one end of the polymer molecule, as well as derivatives of such polymers having esterifiable carboxy groups. Polyethylene glycols of an average molecular weight ranging from 200-20,000 are preferred; those having an average molecular weight ranging from 500-2000 are particularly preferred.

The use of terms "a" and "an" and "the" and similar referents in the context of describing the invention (especially in the context of the following claims) are to be construed to cover both the singular and the plural, unless otherwise indicated herein or clearly contradicted by context. The terms "comprising", "including", "having", and "containing" are to be construed as open-ended terms (i.e. meaning "including but not limited to") unless otherwise noted. The use of any and all examples, or exemplary language (e.g., "such as") provided herein, is intended merely to better illuminate the invention and does not pose a limitation on the scope of the invention unless otherwise claimed. No language in the specifications should be construed as indicating any non-claimed element as essential to the practice of the invention.

### DETAILED DESCRIPTION OF THE INVENTION

This invention relates to the preparation of lipid formulations as set out in the claims.

In particular, the present invention relates to composition and method of preparing organic solvent-free formulation comprising one or more active compounds.

The present invention also relates to compositions and methods of delivering anticancer drugs, for example, docetaxel and paclitaxel, and immunosuppressant agents, such as tacrolimus and sacrolimus.

The present invention relates to compositions and methods for delivering polyene antibiotics that reduce the toxicity of the antibiotic to the host being treated.
Several formulation strategies have been used to reduce the nephrotoxicity of amphotericin

Amphotericin B is insoluble in aqueous solution and before it can be used clinically as an antifungal agent, a vehicle (carrier) has to be added to form dispersion. The commercial preparation of amphotericin B, Fungizone^{®} is a mixture of amphotericin B, a detergent deoxycholate, and a buffer. When suspended in a glucose solution, Fungizone^{®} forms colloidal dispersion suitable for intravenous injection. (Brajtburg, J. et al. 1990). Fungizone^{®}, the first marketed formulation of amphotericin B with deoxycholate remains the gold standard in spite of its renal toxicity. Fungizone^{®} is currently marketed as lyophilized cake providing 50 mg amphotericin B and 41 mg of deoxycholate with 20.2 mg of sodium phosphates as a buffer.

In an effort to improve the delivery of amphotericin B in the treatment of fungal diseases, several liposome formulations have been designed. Liposomal composition containing egg phosphatidylcholine, dipalmitoyl phosphatidylethanolamine, and cholesterol in molar ratio of 6:1:3 were more efficient in improving the therapeutic index as compared to free drug. Further, amphotericin B intercalated into mannosylated liposomes is less toxic and more effective as fungal killer (Ahmad, I. et al., 1989, 1990, 1991).

AmBisome^{®} is a lyophilized formulation of amphotericin B incorporated into unilamellar liposomes formed from soy phosphatidylcholine, distearoylphosphatidylglycerol, and cholesterol. AmBisome^{®} binds to the fungal cells, resulting in death of the fungus. (Adler-Moore, Jill P. et al., 1994; Adler-Moore et al. 1993). AmBisome^{®} formulation has greatly reduced the toxicity of amphotericin B, and high plasma concentrations and tissue accumulations of drug can be achieved with non-toxic doses of AmBisome^{®} (Proffitt etal, US 5,965,156, 1999; Proffitt, R. T. 1991).

Abelcet^{®} is liposome formulation consists of a 1:1 ratio of amphotericin B in combination with a 7:3 ratio of dimyristoyl phosphatidylcholine to dimyristoyl phosphatidylglycerol. The resulting complex forms a tightly packed ribbon structure, approximately 250 nm diameter. The safety and efficacy of Abelcet^{®} have been extensively evaluated in clinical studies and have shown that Abelcet^{®} is, in general less toxic than amphotericin B deoxycholate (Lister, J. 1996; Walsh, T.J. et al 1997).

In order to reduce the toxicity of amphotericin B, a new formulation has been developed consisting of a cholesteryl sulfate complex with amphotericin B, the amphotericin B colloidal dispersion (Amphotec^{®}). Amphotec^{®} is a stable complex of amphotericin B and cholesteryl sulfate in a 1:1 molar ratio. In vitro studies with fresh human blood have shown that the drug-lipid complex does not result in hemolysis of erythrocytes and that binding to plasma lipoproteins is less than that observed with Fungi zone. However, the pharmacokinetics of amphotericin B following infusions of ABCD does not differ significantly from those of Fungizone^{®}. (Szoka, F.C. Jr. US 5277914 A 1994; Abra, R. and Guo, L.S. US 5,194,266, 1993; Abra, R. US5,032,582, 1991; Abra, R. US4,822,777, 1989; Abra, R. et al. PCT Appl WO8701933, 1987; Sanders, S. et al. 1991).

The various lipid formulations of amphotericin B described above, however, are still capable of producing all of the toxicities associated with amphotericin B alone, although nephrotoxicity is reduced to some extent with all these formulations.

The present invention provides formulations using new lipid compositions that reduce the toxicities associated with active compounds such as amphotericin B deoxycholate.

The present invention provides compositions and methods for delivering active compounds such as polyene antibiotics, *e*.*g*., to a mammalian host. Examples of polyene antibiotics that find use in the present invention include but are not limited to amphotericin B deoxycholate (Fungizone^{®}), Nystatin (Nys), Natamycin, Candicidin, Aureofungin A, Aureofungin B, Hamycin A, Hamycin B, Trienin, Pimaricin, Etruscomycin, Chainin, Dermostatin, Filipin, and Lymphosarcin. In some preferred embodiments, the present invention comprises compositions and methods for the delivery of amphotericin B deoxycholate (Fungizone^{®}) to a mammalian host. Any suitable amount of an active compound, *e*.*g*., polyene antibiotics such as amphotericin B deoxycholate, can be used. Suitable amounts of polyene antibiotic are those amounts that can be stably incorporated into the complexes of the present invention.

The present invention provides compositions and methods of delivering anticancer drugs, e.g., to a mammalian host. Examples of anticancer drugs that find use in the present invention include but are not limited to paclitaxel, docetaxel, doxorubicin, daunomycin, epirubicin, etoposide, tamoxifen, endoxifen, vincristine anthracycline, and the like. Any suitable amount of anticancer drugs can be used. Suitable amounts of anticancer drugs are those amounts that can be stably incorporated into the complexes of the present invention.

The present invention provides compositions and method of delivering immunosuppressant agents. Examples of immunosuppressant agents that find use in the present invention include but not limited to tacrolimus and sacrolimus. Any suitable amount of immunosuppressant agents can be used. Suitable amounts of immunosuppressant agents are those amounts that can be incorporated into the complexes of the present invention.

The present inventions provide compositions and method for treating rejection reactions caused by the transplantations organs and tissues. Examples of organs and tissue transplantation include but not limited to heart, kidney, liver, lung, bone marrow, skin, cornea, pancreas, small intestine, muscle, limb, myoblast, intervertebral disc, cartilage, bone, blood vessel, nervous system, esophagus and the like.

In some embodiments, the present description comprises a lipid complex with active compound (for example, amphotericin B with or without deoxycholate) in which the complex contains lipid or a mixture of lipids. In some embodiments, the complexes are in the form of micelles, emulsions or mixture of micelles and vesicles. The micelles of the present invention can be in the form, e.g., of monomeric, dimeric, polymeric or mixed micelles. In some embodiments, the complexes including micelles, emulsions or mixture of micelles and vesicles are predominately in the size range of 50nm-20 micron, while in some preferred embodiments, the micelles and emulsions are in the size range of 50nm-5 micron. In the complexes of the present invention, the antibiotic can be bound to the lipid by covalent, hydrophobic, electrostatic, hydrogen, or other bonds, and is considered "bound" even where the antibiotic is simply entrapped within the interior of lipid.

Active agent-lipid complexes (for example, amphotericin B-lipid complexes with or without deoxycholate) contain cholesterol or cholesterol derivatives. Examples of cholesterol derivatives that find use in the present invention include but are not limited to cholesteryl sulfate, cholesteryl hemisuccinate.

In some preferred embodiments, the compositions also include α-, β-, γ-tocopherols, vitamin E, calciferol, organic acid derivatives of α-, β-, γ-tocopherols, such as α-tocopherol hemisuccinate (THS), α-tocopherol succinate, or mixtures thereof.

In some preferred embodiments, active agent-lipid complexes (for example, amphotericin B-lipid complexes, with or without deoxycholate) contain sterols. Examples of sterols that find use in the present invention include β-sitosterol, stigmasterol, stigmastanol, lanosterol, α-spinasterol, lathosterol, campesterol and/or mixtures thereof.

Compositions of the present description also include active compounds (for example, amphotericin B complexes with or without deoxycholate) with free and/or salts or esters of fatty acid. In some preferred embodiments, fatty acids range from carbon chain lengths of about C₂ to C₃₄, preferably between about C₄ and about C₂₄, and include tetranoic acid (C₄:o), pentanoic acid (C_{5:}o), hexanoic acid (C_{6:0}), heptanoic acid (C_{7:0}), octanoic acid (C_{8:0}), nonanoic acid (C_{9:0}), decanoic acid (C_{10:0}), undecanoic acid (C_{11:0}), dodecanoic acid (C_{12:0}), tridecanoic acid (C_{13:0}), tetradecanoic (myristic) acid (C_{14:0}), pentadecanoic acid (C_{15:0}), hexadecanoic (palmatic) acid (C_{16:0}), heptadecanoic acid (C_{17:0}), octadecanoic (stearic) acid (C_{18:0}), nonadecanoic acid (C_{19:0}), eicosanoic (arachidic) acid (C₂o:o), heneicosanoic acid (C_{21:0}), docosanoic (behenic) acid (C_{22:}o), tricosanoic acid (C_{23:o}), tetracosanoic acid (C_{24:}o), 10-undecenoic acid (C_{11:1}), 11-dodecenoic acid (C_{12:1}), 12-tridecenoic acid (C_{13:1}), myristoleic acid (C_{14:1}), 10-pentadecenoic acid (C_{15:1}), palmitoleic acid (C_{16:1}), oleic acid (C_{18:1}), linoleic acid (C_{18:2}), linolenic acid (C_{18:3}), eicosenoic acid (C_{20:1}), eicosdienoic acid (C_{20:2}), eicosatrienoic acid (C_{20:3}), arachidonic acid (*cis*-5,8,11,14-eicosatetraenoic acid), and *cis*-5,8,11,14,17-eicosapentaenoic acid, among others. Other fatty acid chains also can be employed in the compositions. Examples of such include saturated fatty acids such as ethanoic (or acetic) acid, propanoic (or propionic) acid, butanoic (or butyric) acid, hexacosanoic (or cerotic) acid, octacosanoic (or montanic) acid, triacontanoic (or melissic) acid, dotriacontanoic (or lacceroic) acid, tetratriacontanoic (or gheddic) acid, pentatriacontanoic (or ceroplastic) acid, and the like; monoethenoic unsaturated fatty acids such as *trans*-2-butenoic (or crotonic) acid, *cis*-2-butenoic (or isocrotonoic) acid, 2-hexenoic (or isohydrosorbic) acid, 4-decanoic (or obtusilic) acid, 9-decanoic (or caproleic) acid, 4-dodecenoic ( or linderic) acid, 5-dodecenoic (or denticetic) acid, 9-dodecenoic (or lauroleic) acid, 4-tetradecenoic (or tsuzuic) acid, 5-tetradecenoic (or physeteric) acid, 6-octadecenoic (or petroselenic) acid, *trans*-9-octadecenoic ( or elaidic) acid, *trans-*11-octadecenoic ( or vaccinic) acid, 9-eicosenoic ( or gadoleic) acid, 11-eicosenoic ( or gondoic) acid, 11-docosenoic ( or cetoleic) acid, 13-decosenoic ( or erucic) acid, 15-tetracosenoic ( or nervonic) acid, 17-hexacosenoic ( or ximenic) acid, 21-triacontenoic ( or lumequeic) acid, and the like; dienoic unsaturated fatty acids such as 2,4-pentadienoic (or β-vinylacrylic) acid, 2,4-hexadienoic (or sorbic) acid, 2,4-decadienoic (or stillingic) acid, 2,4-dodecadienoic acid, 9,12-hexadecadienoic acid, *cis-*9, *cis-*12-octadecadienoic (or α-linoleic) acid, *trans-*9, *trans*-12-octadecadienoic (or linlolelaidic) acid, t*rans*-10,*trans*-12-octadecadienoic acid, 11,14-eicosadienoic acid, 13,16-docosadienoic acid, 17,20-hexacosadienoic acid and the like; trienoic unsaturated fatty acids such as 6,10,14-hexadecatrienoic (or hiragonic) acid, 7,10,13-hexadecatrienoic acid, *cis-*6, *cis-9- cis*-12-octadecatrienoic (or γ-linoleic) acid, *trans*-8, *trans-*10*- trans*-12-octadecatrienoic (or β-calendic) acid, *cis-*8*, trans-*10*- cis*-12-octadecatrienoic acid, *cis-*9, *cis-*12*- cis*-15-octadecatrienoic (or α-linolenic) acid, *trans*-9, *trans-*12- *trans-15-*octadecatrienoic (or α-linolenelaidic) acid, *cis-*9, *trans-*11*- trans*-13-octadecatrienoic (or α-eleostearic) acid, *trans*-9, *trans-*11- *trans*-13-octadecatrienoic (or β-eleostearic) acid, *cis-*9, *trans-*11- *cis*-13-octadecatrienoic (or punicic) acid, 5,8,11-eicosatrienoic acid, 8,11,14-eicosatrienoic acid and the like; tetraenoic unsaturated fatty acids such as 4,8,11,14-hexadecatetraenoic acid, 6,9,12,15- hexadecatetraenoic acid, 4,8,12,15-octadecatetraenoic (or moroctic) acid, 6,9,12,15- octadecatetraenoic acid, 9,11,13,15- octadecatetraenoic (or α -or β-parinaric) acid, 9,12,15,18-octadecatetraenoic acid, 4,8,12,16-eicosatetraenoic acid, 6,10,14,18-eicosatetraenoic acid, 4,7,10,13-docasatetraenoic acid, 7,10,13,16-docosatetraenoic acid, 8,12,16,19-docosatetraenoic acid and the like; penta- and hexa-enoic unsaturated fatty acids such as 4,8,12,15,18-eicosapentaenoic (or timnodonic) acid, 4,7,10,13,16-docosapentaenoic acid, 4,8,12,15,19-docosapentaenoic (or clupanodonic) acid, 7,10,13,16,19-docosapentaenoic, 4,7,10, 13,16,19-docosahexaenoic acid, 4,8,12,15,18,21-tetracosahexaenoic (or nisinic) acid and the like; branched-chain fatty acids such as 3-methylbutanoic (or isovaleric) acid, 8-methyldodecanoic acid, 10-methylundecanoic (or isolauric) acid, 11-methyldodecanoic (or isoundecylic) acid, 12-methyltridecanoic (or isomyristic) acid, 13-methyltetradecanoic (or isopentadecylic) acid, 14-methylpentadecanoic (or isopalmitic) acid, 15-methylhexadecanoic, 10-methylheptadecanoic acid, 16-methylheptadecanoic (or isostearic) acid, 18-methylnonadecanoic (or isoarachidic) acid, 20-methylheneicosanoic (or isobehenic) acid, 22-methyltricosanoic (or isolignoceric) acid, 24-methylpentacosanoic (or isocerotic) acid, 26-methylheptacosanoic (or isomonatonic) acid, 2,4,6-trimethyloctacosanoic (or mycoceranic or mycoserosic) acid, 2-methyl-*cis*-2-butenoic(angelic)acid, 2-methyl-*trans-2-*butenoic (or tiglic) acid, 4-methyl-3-pentenoic (or pyroterebic) acid and the like.

In certain preferred embodiments, active compounds (for example, amphotericin B-lipid complexes with or without deoxycholate) comprise phospholipids. Any suitable phospholipids can be used. For example, phospholipids can be obtained from natural sources or chemically synthesized. Examples of phospholipids that find use in the present invention include phosphatidylethanolamine (PE), phosphatidylglycerol (PG), phosphatidylserine (PS), phosphatidylcholine (PC), phosphatidylinositol (PI), phosphatidic acid (PA), sphingomyelin and the like, either used separately or in combination. Phosphatidylglycerols may be having short chain or long chain, saturated or unsaturated such as dimyristoylphosphatidylglycerol, dioleoylphosphatidylglycerol, distearoylphosphatidylglycerol, dipalmitoylphosphatidylglycerol, diarachidonoylphosphatidylglycerol, short chain phosphatidylglycerol (C₆-C₈), and mixtures thereof. Examples of phosphatidylcholines includes dimyristoylphophatidylcholine, distearoylphosphatidylcholine, dipalmitoylphosphatidylcholine, dioleoylphosphatidylcholine, diarachidonoylphosphatidylcholine, egg phosphatidylcholine, soy phosphatidylcholine or hydrogenated soy phosphatidylcholine can be used, as can mixtures thereof.

In some embodiments, the present description provides compositions comprising at least one active compound (for example, amphotericin B with or without deoxycholate) and derivatives of mono-, di- and tri-glycerides. Examples of the glycerides that find use in the present invention include but are not limited to 1-oleoyl-glycerol (monoolein) and 1, 2-dioc-tanoyl-5/7-glycerol.

Another aspect of the invention is to complex at least one active compound (for example, amphotericin B with or without deoxycholate) with at least one functionalized phospholipid, including but not limited to phosphatidylethanolamine, phosphatidylthioethanol, N-biotinylphosphatidylethanolamine, and phosphatidylethylene glycol. In some preferred embodiments, amphotericin B with or without deoxycholate is complexed with dioleoylphosphatidylethanolamine.

Another aspect of the description is to complex at least one active compound (for example, amphotericin B with or without deoxycholate) with at least one carbohydrate-based lipid. Examples of carbohydrate-based lipids that find use in the present invention include but are not limited to galacto lipids, manno lipids, galactolecithin and the like.

Yet another aspect of the description is to complex at least one active compound (for example, amphotericin B with or without deoxycholate) with derivatives of phospholipids such as pegylated phospholipids. Examples include but not limited to the polyethylene glycol (Pegylated, PEG) derivatives of distearoylphosphatidylglycerol, dimyristoylphosphatidylglycerol, dioleoylphosphatidylglycerol and the like.

Another further aspect of the present invention provides compositions comprising at least one active compound (for example, amphotericin B with or without deoxycholate) and polyethylene glycol (PEG) and one or more lipids.

According to another aspect, the present description provides compositions comprising at least one active compound (for example, amphotericin B with or without deoxycholate) complexed with one or more lipids. Example includes compositions comprising amphotericin B with or without deoxycholate, cholesterol or cholesterol derivatives and one or more phospholipids. Other examples of compositions according to the description include amphotericin B with or without deoxycholate, β-sitosterol, and one or more phospholipids. In some preferred embodiments, the composition of the present invention comprises amphotericin B, with or without deoxycholate, cholesteryl sulfate and hydrogenated soy phosphatidylcholine or soy phosphatidylcholine.

The composition of the present invention can be made by dissolving an active compound, for example, amphotericin B deoxycholate (e.g., Fungizone^{®}) in water at a concentration of about 0.5 mg/mL to about 25 mg/mL. In some embodiments, the antibiotic is dissolved at a concentration between 1 mg/mL and about 20 mg/mL. In certain preferred embodiments, the antibiotic is dissolved at a concentration of between 1 mg/mL and 10 mg/mL. In particularly preferred embodiments, the antibiotic is dissolved at a concentration of between 1 mg/mL and 5 mg/mL.

In some embodiments, compositions of the present invention contain about 2.5% to about 95% by weight of total lipid, preferably about 10% to about 90% by weight of total lipid or more, preferably about 20% to about 90% by weight of total lipid.

In some embodiments, compositions of the present invention contain at least one active compound (for example, amphotericin B, with or without sodium deoxycholate) and lipid(s) in mole ratio between 1 :1 to 1 :100, e.g. , in between 1 : 1 and 1 :20 molar ratio or in between 1 : 1 and 1 :30 molar ratio or in between 1 : 1 and 1 :40 molar ratio or in between 1 : 1 and 1 :50 molar ratio, in between 1 : 1 and 1 :60 molar ratio, in between 1 : 1 and 1 :70 molar ratios, and in between 1 : 1 and 1 :80 molar ratios. As used herein, the term "in between" is inclusive of the limits of a recited range. For example, a mole ratio "in between" 1 : 1 and 1 :20 molar ratio includes ratios of 1 : 1 and 1 :20.

In certain preferred embodiments, compositions of the present invention contain at least one active compound (for example, amphotericin B, with or without sodium deoxycholate), cholesteryl sulfate and hydrogenated soy phosphatidylcholine. Such compositions include amphotericin B and sodium deoxycholate in mole ratio of 1 :2.

In certain preferred embodiments, the mole ratio of active compound (for example, amphotericin B) and cholesteryl sulfate in a composition containing active compound (for example, amphotericin B), sodium deoxycholate, cholesteryl sulfate and hydrogenated soy phosphatidylcholine is in between 1:1 and 1:20, such as in between 1:1 and 1:10, or in between 1:1 and 1:5 or 1:1 and 1:2. In particularly preferred embodiments, the mole ratio of active compound (for example, amphotericin B) and cholesteryl sulfate is in between 1:1 and 1:5.

In certain preferred embodiments, the mole ratio of active compound (for example, amphotericin B) and hydrogenated soy phosphatidylcholine in a composition containing active compound (for example, amphotericin B, with or without sodium deoxycholate), cholesteryl sulfate and hydrogenated soy phosphatidylcholine is in between about 1:1 and 1:90, *e*.*g*., in between 1:1 and 1:70 or 1:1 and 1:60 or 1:1 and 1:50 or 1:1 and 1:40 and 1:1 and 1:30. In particularly preferred embodiments, the mole ratio of active compound (for example, amphotericin B) and hydrogenated soy phosphatidylcholine is in between 1:5 and 1:60.

In certain preferred embodiments, the mole ratio of active compound (for example, amphotericin B) and soy phosphatidylcholine in a composition containing active compound (for example, amphotericin B), with or without sodium deoxycholate, cholesteryl sulfate and soy phosphatidylcholine is in between 1:1 and 1:90, *e*.*g*., in between 1:1 and 1:70 or 1:1 and 1:60 or 1:1 and 1:50 or 1:1 and 1:40 and 1:1 and 1:30. In particularly preferred embodiments, the mole ratio of active compound (for example, amphotericin B) and soy phosphatidylcholine is in between 1:5 and 1:60.

In some embodiments, compositions of the present invention contain active compound (for example, amphotericin B) and total lipids having weight-to-weight ratio between 1:1 to 1:100 ratio such as in between 1:1 and 1:20 ratio or in between 1:1 and 1:30 ratio or in between 1:1 and 1:40 ratio or in between 1:1 and 1:50 ratio, or in between 1:1 and 1:60 ratio, or in between 1:1 and 1:70 ratio, and in between 1:1 and 1:80 ratio, or in between 1:1 and 1:90 ratio.

In some embodiments, the mole ratio of cholesterol or cholesteryl derivative (such as cholesteryl sulfate) and one or more phospholipids (for example, soy phosphatidylcholine) is in between 1:1 and 1:90, *e*.*g*., in between 1:1 and 1:70 or 1:1 and 1:60 or 1:1 and 1:50 or 1:1 and 1:40 and 1:1 and 1:30. In particularly preferred embodiments, the mole ratio of cholesterol derivative (for example, cholesteryl sulfate) and soy phosphatidylcholine is in between 1:1 and 1:20.

In some embodiments, the methods of the present invention involve dissolving active compound, *e*.*g*., amphotericin B (with or without deoxycholate), in water and mixing the dissolved antibiotic and the lipid(s) together. The active compound-lipid complex solution can be filtered through suitable filters to control the size distribution of the formed complexes.

In some embodiments, the method of the present invention involves mixing lipid(s) and sodium deoxycholate together in water and then adding active compound (for example, amphotericin B). The active compound-lipid complex solution can be filtered through suitable filters to control the size distribution of the formed complexes.

In some embodiments, the method comprises mixing amphotericin B and cholesteryl derivative, for example cholesteryl sulfate in water or buffer having pH in the range of 1 to 3.0 and can be heated if desired at temperature ranging from 25° C to 60° C. The resulting suspension is then mixed with phospholipids, for example soy phoaphatidylcholine or hydrogenated soy phosphatidylcholine in water or buffer and the pH is adjusted with suitable base or buffer so the resulting suspension attains a pH ranging between 5.00 and 8.00. The acidic pH can be achieved by any suitable acid such as hydrochloric acid, phosphoric acid and the like. Examples of base or buffer includes but not limited to sodium succinate dibasic, sodium acetate, sodium phosphate monobasic, sodium phosphate dibasic, sodium phosphate tribasic, sodium hydroxide, and the like. The composition may further contain sugar. Examples of sugars includes but not limited to sucrose, lactose, dextrose, trehalose maltose, and the like. The percentage of sugar may range from 5% to about 25%. The resulting suspension can be homogenized or sonicated to reduce the particle size. In some embodiments, the hydrated suspension is filtered through suitable filters to control the size distribution of the formed complexes. In some embodiments, the hydrated composition can be lyophilized to obtain the composition in powder form. In some embodiments, the hydrated composition can be autoclaved.

In some embodiments, the present invention comprises mixing amphotericin B, sodium deoxycholate, and one or more lipids in any suitable sequence such that the resulting composition of the present invention comprises amphotericin B, sodium deoxycholate and one or more lipids. For example, in some embodiments, the method comprises of mixing amphotericin B in a solution containing sodium deoxycholate in water and then adjusting the pH with sodium hydroxide until the amphotericin B is completely dissolved. Lipids such as soy phosphatidylcholine are then added to the amphotericin B-sodium deoxycholate solution, followed by one more lipid, such as cholesteryl sulfate. The amphotericin B -lipid complex solution can be filtered through suitable filters to control the size distribution of the formed complexes.

In some embodiments, the present invention comprises mixing active compound (for example, amphotericin B), and one or more lipids in any suitable sequence such that the resulting composition of the present invention comprises active compound (for example, amphotericin B), and one or more lipids. For example, in some embodiments, the method comprises of mixing amphotericin B in water and then adjusting the pH with sodium hydroxide until the amphotericin B is completely dissolved. Lipids such as soy phosphatidylcholine are then added to the amphotericin B solution, followed by one more lipid, such as cholesteryl sulfate. The amphotericin B -lipid complex solution can be filtered through suitable filters to control the size distribution of the formed complexes. In another embodiment the amphotericin B and cholesteryl sulfate is mixed at any desired pH such as at low pH for example pH in between 1.00 and 4.00 or at higher pH for example, pH in between 9.00 and 12.00. The pH is then adjusted with suitable base or buffer to attain the pH of the resulting suspension in the range between 4.00 to 8.00 and then mixed with phospholipids, for example soy phosphatidylcholine or hydrogenated phosphatidylcholine.

In some embodiments, the method of preparation of the present invention comprises heating a composition comprising active compound (for example, amphotericin B in water) with or without deoxycholate and one or more lipids. In some embodiments, heating is at temperatures ranging from 30-121° C. In some preferred embodiments, heating is at a temperature between 40 - 80° C, while in some particularly preferred embodiments, heating is at a temperature between 40-70° C. In some embodiments, the hydrated composition can be autoclaved.

In some embodiments, the method of preparation of present invention comprising mixing active compound (for example, Tacrolimus), cholesteryl derivative (for example, cholesteryl sulfate) and phosphatidylcholine such as soy phosphatidylcholine or hydrogenated soy phosphatidylcholine in water or buffer. The resulting suspension can be homogenized or sonicated at any desired temperature ranging from 20-60°C. Examples of base or buffer includes but not limited to sodium succinate dibasic, sodium acetate, sodium phosphate monobasic, sodium phosphate dibasic, sodium phosphate tribasic, sodium hydroxide, and the like. The composition may further contain sugar. Examples of sugars includes but not limited to sucrose, lactose, dextrose, trehalose, maltose, and the like. The percentage of sugar may range from 5% to about 25%. The resulting suspension can be homogenized or sonicated to reduce the particle size. In some embodiments, the hydrated suspension is filtered through suitable filters to control the size distribution of the formed complexes. In some composition, the hydrated suspension can be lyophilized to obtain the composition in powder form. In some embodiments, the hydrated composition can be autoclaved.

In some embodiments, the method of preparation of present invention comprising mixing active compound (for example, Docetaxel), cholesteryl derivative (for example, cholesteryl sulfate) and phosphatidylcholine such as soy phosphatidylcholine or hydrogenated soy phosphatidylcholine in water or buffer. The resulting suspension can be homogenized or sonicated at any desired temperature ranging from 20-120°C. Examples of base or buffer includes but not limited to sodium succinate dibasic, sodium acetate sodium phosphate monobasic, sodium phosphate dibasic, sodium phosphate tribasic, sodium hydroxide, and the like. The composition may further contain sugar. Examples of sugars includes but not limited to sucrose, lactose, dextrose, trehalose, maltose, and the like. The percentage of sugar may range from 5% to about 25%. The resulting suspension can be homogenized or sonicated to reduce the particle size. In some embodiments, the hydrated suspension is filtered through suitable filters to control the size distribution of the formed complexes. In some composition, the hydrated suspension can be lyophilized to obtain the composition in powder form. In some embodiments, the hydrated composition can be autoclaved

In some embodiments, the method of preparation of present invention comprising mixing active compound (for example, Paclitaxel), cholesteryl derivative (for example, cholesteryl sulfate) and phosphatidylcholine such as soy phosphatidylcholine or hydrogenated soy phosphatidylcholine in water or buffer. The resulting suspension can be homogenized or sonicated at any desired temperature ranging from 20-120°C. Examples of base or buffer includes but not limited to sodium succinate dibasic, sodium acetate, sodium phosphate monobasic, sodium phosphate dibasic, sodium phosphate tribasic, sodium hydroxide, and the like. The composition may further contain sugar. Examples of sugars includes but not limited to sucrose, lactose, dextrose, trehalose, maltose, and the like. The percentage of sugar may range from 5% to about 25%. The resulting suspension can be homogenized or sonicated to reduce the particle size. In some embodiments, the hydrated suspension is filtered through suitable filters to control the size distribution of the formed complexes. In some composition, the hydrated suspension can be lyophilized to obtain the composition in powder form. In some embodiments, the hydrated composition can be autoclaved.

The pH of the composition of invention ranges from about 4.0 to pH 8.0. In some embodiments, aqueous solutions having suitable pH are prepared from water having appropriate amount of buffers dissolved in it. In some preferred embodiments, buffers comprise mixtures of monobasic sodium phosphate, dibasic sodium phosphate and tribasic sodium phosphate. In some preferred embodiments, buffers comprise sodium carbonate, sodium bicarbonate, sodium hydroxide, ammonium acetate, sodium succinate, sodium citrate, tris (hydroxy-methyl) aminoethane, sodium benzoate, sodium acetate, and the like.

In some embodiments, filters are used to obtain the desired size range of the complexes from the filtrate. For example, the complexes can be formed and thereafter filtered through a 5 micron filter to obtain complex having a diameter of about 5 micron or less. Alternatively, 1 µm, 500 nm, 200 nm, 100 nm or other filters can be used to obtain complexes having diameters of about 1 µm, 500 nm, 200 nm, 100 nm or any suitable size range, respectively.

In some embodiments, the composition of the present invention can be sterilized by filtering through 0.22 µm or 0.45 µm filter under aseptic conditions. In another embodiments, the composition of the present invention can be sterilized by autoclaving in the range of 120°C-130°C for a duration of 15-20 minutes.

In some embodiments, the active compound-lipid complex (for example, amphotericin B-lipid complex) with or without deoxycholate is dried, e.g., by evaporation or lyophilization. In certain embodiments of the invention, the active compound-lipid complex (for example, amphotericin B-lipid complex) with or without deoxycholate is lyophilized with one or more cryo-protectants, such as sugars. Examples of sugars that find use in the present invention include but are not limited to trehalose, maltose, lactose, sucrose, glucose, and dextran. In preferred embodiments, the compositions of the present invention comprise trehalose and/or sucrose. The lyophilization is generally accomplished under vacuum and can take place either with or without prior freezing of the active compound-lipid complex (for example, amphotericin B-lipid preparation) with or without deoxycholate. While not limiting the lyophilization of the present invention to any particular configuration, the lyophilization in the present invention can be done, e.g., in vials or other containers having desired volumes. The lyophilization can also be done as bulk in trays. When desired, the complexes can be resuspended in any desirable solvent including water, saline, dextrose and buffer.

Pharmaceutical preparations that find use in the present description include but are not limited to tablets, capsules, solutions, suspensions, emulsions, ointments; gels can be suitable pharmaceutical preparations. In some embodiments, e.g., for the oral mode of administration, active compound-lipid complex (for example, amphotericin B-lipid complex, tacrolimus lipid complex, paclitaxel or docetaxel lipid complexes) with or without deoxycholate is used in the form of tablets, capsules, lozenges, powders, syrups, aqueous solutions, suspensions and the like. In some embodiments, e.g., for topical application, active compound-lipid complex (for example, amphotericin B-lipid complex, with or without deoxycholate) is provided in the form of gels, oils, and emulsions, such as are known by the addition of suitable water-soluble or water-insoluble excipients, for example polyethylene glycols, certain fats, and esters, compounds having a higher content of polyunsaturated fatty acids and derivatives thereof. Derivatives include but are not limited to mono-, di-, and triglycerides and their aliphatic esters (for example, fish oils, vegetable oils etc.) or mixtures of these substances. In some embodiments, excipients that find use in conjunction with the compositions of the present invention comprise those in which the drug complexes are sufficiently stable to allow for therapeutic use.

In some embodiments, preparations of active compound-lipid complex (for example, amphotericin B-lipid complex with or without deoxycholate or tacrolimus-lipid complex, paclitaxel or docetaxel lipid complexes) are prepared in enteric coated tablets or capsules, *e*.*g*., to protect it from acids in the stomach. "Enteric" refers to the small intestine, therefore "enteric coating" generally refers to a coating that substantially prevents release of a medication before it reaches the small intestine. While not limiting the invention to any particular mechanism of action, it is understood that most enteric coatings work by presenting a surface that is stable at acidic pH but breaks down rapidly at higher pH. Enteric coatings that find use in the present invention comprise capsules filled with active compound-lipid complex (for example, amphotericin B-lipid complex with or without deoxycholate, tacrolimus lipid complex, paclitaxel or docetaxel lipid complexes) as according to methods well known in the art.

Preparations of active compound-lipid complex (for example, amphotericin B-lipid complex) with or without deoxycholate of the present invention can comprise complexes of varying size, or can comprise complexes of substantially uniform size. For example, in some embodiments the complexes have a size range of about 1 mm or less, while in preferred embodiments, the complexes are in the micron or sub-micron range. In some embodiments, the complexes have a diameter of about 5 µm or less, such as 0.2 µm or less, or even 0.1 µm or less.

Active compound-lipid complex (for example, amphotericin B-lipid complex, with or without deoxycholate) of the present invention may comprise or consist essentially of micelles, mixed micelles, liposomes and vesicles of different shape and sizes.

As noted above, the technology outlined in the present invention for the preparation of amphotericin B complexes is also suitable for use with any other water-insoluble drugs.

In some embodiments, the inventive amphotericin B-lipid complex (with or without deoxycholate) is employed to treat a fungal infection, *e*.*g*., in a mammal. In this regard, the invention provides a method of treating fungal infections comprising administering to a subject (*e*.*g*. a patient having a fungal infection) a composition comprising a complex of amphotericin B-with or without deoxycholate and lipid(s) in an amount sufficient to treat the fungal infection within the subject.

The composition of the present invention can be employed to treat infections caused by numerous fungi and parasites, including but not limited to, *Acremonium sp*., *Aspergillus fumigatus*, *Aspergillus pneumonia, Blastomyces dermatitidis*, *Candida albicans, Candida guillermondi, Candida tropicalis, Coccidioides immitis*, *Cryptococcus neoformans*, *Fusarium sp., Histoplasma capsulatum, Mucor mucedo, Rhodotorula sp., Sporothrix schenckii, Acanthamoeba polyphaga, Entomophthora sp., Histoplasma capsulatumm Leishmania brasiliensis, Rhizopus sp., Rhodotorula sp*., *Torulopsis glabrata, Paracoccidioides brasiliensis. Additional fungal pathogens include Trichosporon, Muco, Alternaria, Bipolaris, Curvularia, etc.*

The composition of present invention can also be employed to treat Visceral Leishmaniasis also called as Kala-azar and infections caused by *Leishmania donovani complex, L.d donovani, L.d infantum*, *L.d archibaldi, L.d chagasi, Phlebotomus sp. and Lutzomya logipalpis.*

The composition of present invention can also be employed to treat viral infections such as those caused, *e*.*g*., by human immunodeficiency virus (HIV), herpes simplex viruses (HSV-1 and HSV2), hepatitis C virus (HCV) and cyotomegalovirus (CMV).

In some embodiments, the inventive active compound lipid-complex (for example, docetaxel-lipid complex or paclitaxel-lipid complex) is employed to treat a cancer, e.g., in a mammal. In this regard, the invention provides a method of treating cancer comprising administering to a subject (e.g. a patient having a cancer) a composition comprising a complex of active compound lipid-complex (for example, docetaxel-lipid complex or paclitaxel-lipid complex) and lipid(s) in an amount sufficient to treat the cancer within the subject. The cancer can be any type of cancer in a mammal. Examples include, but are not limited to cancers of the head, neck, brain, blood, (e.g. leukemia, acute leukemia, acute lymphocytic leukemia, acute myelocytic leukemia, lymphoma, myeloma), breast, lung, pancreas, bone, spleen, bladder, prostate, testes, colon, kidney, ovary and skin (e.g. Kaposi's sarcoma), bone marrow, liver, stomach, tongue, mouth and larynx. In addition, active compound- lipid complex of the present invention are useful in reducing the tendency of cancer cells to develop a resistance to other therapeutic agents such as anticancer agents, chemotherapy and radiation. Thus, other therapeutic agents can be advantageously employed with the present invention in the formation of an active combination or by separate administration.

In some embodiments, the inventive active compound lipid-complex (for example, tacrolimus-lipid complex) is employed to treat rejection reactions caused by organ transplantations and can be administered organ or tissue transplantation, e.g., in a mammal. In this regard, the invention provides a method of preventing organ or tissue rejection comprising administering to a subject (e.g. a patient having an organ or tissue transplantation) a composition comprising a complex of active compound lipid-complex (for example, tacrolimus-lipid complex) and lipid(s) in an amount sufficient to prevent an organ or tissue rejection within the subject.

### EXAMPLE 1

Amphotericin B (1 gm) was suspended in aqueous medium at pH 1.5 to 3.5 and mixed with 3 gm of Sodium Cholesteryl Sulfate. Soya Phosphatidylcholine (7 gm) was stirred and mixed with Amphotericin B and Sodium Cholesteryl Sulfate Complex for 30 min. The mixture was then subjected to high pressure homogenization. The formulation was lyophilized in the presence of 7.5-9.5% sucrose and reconstituted in water for injection. The particle size was determined using Nicomp particle sizer 380. The mean volume diameter amounted to less than 200 nm

### EXAMPLE 2

Amphotericin B formulation with lipids as described in Example I was used to test the hemolysis of red blood cells (RBCs). At 0.16 mg/mL Fungizone^{®} 50% of the cells were lysed compared to Amphotericin B lipid suspension where no lysis occurred after incubation with RBCs. Toxicity study was also carried out in Balb/c mice. A total of 9 mice (7 weeks old) were subjected to intravenous administration of amphotericin B formulation at 20 mg/kg. The mice were monitored for 30 days. At the end of 30 days no mortality was observed. This indicated that maximum tolerated dose using this formulation exceeds 20 mg/kg.

| **Group** | **Dose** | **Survival** |
|---|---|---|
| I | 20 mg/kg | 9/9 |

### EXAMPLE 3

Amphotericin B (1 gm) was suspended in aqueous medium at pH 1.5 to 3.5 and mixed with 3 gm of sodium cholesteryl sulfate. Hydrogenated soya phosphatidylcholine (7 gm) was stirred and mixed with amphotericin B and sodium cholesteryl sulfate complex for 30 min. The mixture was then subjected to high pressure homogenization. The formulation was lyophilized in the presence of 7.5% sucrose and reconstituted in water for injection. The particle size was determined using Nicomp particle sizer 380. The particle size was determined using Nicomp particle sizer 380. The mean volume weighting diameter amounted to less than 200 nm

### EXAMPLE 4

Amphotericin B (20 mg) and sodium deoxycholate (6.56 mg) were dissolved in water (10 mL) at pH 11.00 to 12.5 using sodium hydroxide. The pH was then adjusted to pH 7.00-8.5 with suitable acid (for example, phosphoric acid). Hydrogenated soy phosphatidylcholine (930 mg) and cholesteryl sulfate (10.4 mg) was mixed in water (10 mL) and homogenized or sonicated for 30 minutes. The lipid suspension was then mixed with amphotericin B-deoxycholate solution and further homogenized or sonicated for 1 hr. The suspension can be heated if desired at temperature ranging from 25°C to 60° C. The formulation was lyophilized in the presence of 7.5% sucrose and reconstituted in water for injection. The formulation was tested for toxicity in Balb/c mice and compared with Deoxycholate formulation of Amphotericin B (Fungizone^{®}). The animals were weighed and assigned to different groups randomly (5 animals/group). The results are reported in the table below as the number of mice surviving per total.

| **Treatment** | **Dose (mg/kg)** | **Survival/Total** |
|---|---|---|
| Fungizone^{®} | 0.5 | 5/5 |
| | 1.0 | 5/5 |
| | 2.0 | 4/5 |
| | 4.0 | 0/5 |
| Amphotericin - B Formulation | 12.0 | 5/5 |
| | 14.0 | 5/5 |
| | 17.0 | 5/5 |
| | 20.0 | 0/5 |

The data indicated that the liposome formulation of amphotericin B was significantly less toxic when compared to the marketed product (Fungizone^{®}).

### EXAMPLE 5

Amphotericin B formulation with lipids as described in Example IV was prepared without deoxycholate. The resulting formulation was lyophilized in the presence of 7.5% sucrose or lactose. This formulation also showed similar characteristics as of Example 4.

### EXAMPLE 6

Amphotericin B (50 mg) and Cholesteryl sulfate (50 mg) were mixed together in water at pH 2.5-3. SPC (500 mg) was suspended in water separately which was mixed with amphotericin B and cholesteryl sulfate suspension and homogenized using high pressure homogenizer. The formulation was lyophilized in the presence of 7.5% sucrose and reconstituted in water for injection. The reconstituted formulation was tested for toxicity in Balb/c mice with single dose intravenous injection and no mortality was observed at 20 mg/kg dose level as found in Example II. The particle size was determined using Nicomp particle sizer 380. The particle size data is given in the table below.

| **Mean/Distributions** | **Particle Size (Volume Weighting)** |
|---|---|
| Mean Volume Weighting Diameter | 128.4 nm |
| 99% Distribution | 401.8 nm |
| 90% Distribution | 224.6 nm |
| 80% Distribution | 175.9 nm |
| 75% Distribution | 160.3 nm |
| 50% Distribution | 110.2 nm |
| 25% Distribution | 75.9 nm |

### EXAMPLE 7

Amphotericin B (100 mg) and deoxycholate (33 mg) were dissolved in water at pH 9-12.00 and later adjusted to pH 7.5. The amphotericin B suspension was then mixed with cholesteryl sulfate (52 mg) and hydrogenated soyphosphatidylcholine (4.62 g) in water and sonicated at 60 minutes. The formulation was lyophilized both in vials and in bulk in the presence of 7.5% sucrose and reconstituted in water for injection. The particle size was determined using Nicomp particle sizer 380. The mean volume weighting diameter amounted to less than 200 nm

| **Mean/Distributions** | **Particle Size (Volume Weighting)** |
|---|---|
| Mean Volume Weighting Diameter | 76.1 nm |
| 99% Distribution | 227.1 nm |
| 90% Distribution | 130.2 nm |
| 80% Distribution | 103.1 nm |
| 75% Distribution | 94.3 nm |
| 50% Distribution | 66.0 nm |
| 25% Distribution | 46.2 nm |

### EXAMPLE 8

Amphotericin B (50 mg) and Cholesteryl sulfate (50 mg) are mixed together in sodium succinate buffer at pH 2.5-3. SPC (500 mg) in sodium succinate buffer is suspended in water separately which is mixed with amphotericin B and cholesteryl sulfate suspension and homogenized using high pressure homogenizer. The formulation is lyophilized in the presence of 7.5-9.5% sucrose or 9.5% lactose and reconstituted in water for injection. The particle size was determined using Nicomp particle sizer 380. The mean volume weighting diameter amounted to less than 200 nm

### EXAMPLE 9

Amphotericin B (2 g) and Cholesteryl sulfate (1.04 g) were mixed together in succinate buffer at pH 2.5 and sonicated for 5 min at room temperature. Soy lecithin (18.96 g) in sodium succinate buffer (pH 2.5) was with Amphotericin-Cholesteryl sulfate suspension and homogenized using high pressure homogenizer. The formulation was then autoclaved at 121°C for 15 minutes before it was mixed with 7.5-9.5% sucrose or 9.5% lactose solution under aseptic conditions. The particle size was determined using Nicomp particle sizer 380. The particle size data is shown in the table below.

| **Mean/Distributions** | **Particle Size (Volume Weighting)** |
|---|---|
| Mean Volume Weighting Diameter | 693.3 nm |
| 99% Distribution | 1992.5 nm |
| 90% Distribution | 1169.7 nm |
| 80% Distribution | 934.6 nm |
| 75% Distribution | 858.2 nm |
| 50% Distribution | 608.4 nm |
| 25% Distribution | 431.3 nm |

The HPLC analysis of the inventive formulation comprising amphotericin B, soy phosphatidylcholine, cholesteryl sulfate was done and the results are outlined in the table below.

| **Components** | **Assay Results** |
|---|---|
| Amphotericin B | 96.4% |
| Cholesteryl Sulfate | 95.0% |
| Soy Phosphatidylcholine | 87.5% |

**Systemic Adverse Events:** A comparison between Fungizone^{®} and Amphotericin B Lipid Suspension in Healthy Human Volunteers.

The safety and tolerance of Fungizone^{®} versus Amphotericin B Lipid Suspension was evaluated in Human male subjects. In this study a total 24 volunteers were enrolled. Out of this six ( n=6 ) were given Fungizone^{®} (0.6 mg/kg) intravenously and eighteen (n=18) of them received Amphotericin B Lipid Suspension (0.6mg/kg - 1.5 mg/kg).

In the Amphotericin B Lipid Suspension, mild adverse events were reported in 3/18 (17%) healthy male subjects and 4/6 (66%) who were infused Fungizone^{®}. Overall, Amphotericin B Lipid Suspension is apparently safe and well tolerated upto 1.5 mg / kg.

### EXAMPLE 10

Tacrolimus (20 mg) and Cholesteryl sulfate (20 mg) were mixed in water (10 mL) and sonicated for 30 min to form a suspension. SPC in water (10 mL) was mixed with Tacrolimus and Cholesteryl Sulfate suspension and homogenized using high pressure homogenizer. The formulation was lyophilized both in vials and in bulk in the presence of 7.5% sucrose and reconstituted in water for injection. The particle size was determined using Nicomp particle sizer 380. The mean volume diameter amounted to less than 200 nm.

### EXAMPLE 11

Deoxycholate (1 mg) and Cholesteryl sulfate (1 mg) were mixed in water and sonicated for 30 min to form a suspension. SPC in water was mixed with Tacrolimus and Cholesteryl Sulfate suspension and homogenized using high pressure homogenizer. The formulation was lyophilized in the presence of 7.5-% sucrose and reconstituted in water for injection. The particle size was determined using Nicomp particle sizer 380. The mean volume diameter amounted to less than 200 nm.

### EXAMPLE 12

Tacrolimus (100 mg), Cholesteryl sulfate (60 mg), and Soy lecithin (3.94 g) were mixed together in water (70 mL) and homogenized using high pressure homogenizer. The resulting suspension was then filtered through 0.2 µ filter and then mixed with 7.5% sucrose solution (30 mL) and lyophilized both in vials and in bulk. The particle size was determined using Nicomp particle sizer 380. The mean volume weighting diameter amounted to less than 200 nm.

| **Mean/Distributions** | **Particle Size (Volume Weighting)** |
|---|---|
| Mean Volume Weighting Diameter | 42.9 nm |
| 99% Distribution | 141.0 nm |
| 90% Distribution | 76.5 nm |
| 80% Distribution | 59.2 nm |
| 75% Distribution | 53.8 nm |
| 50% Distribution | 36.5 nm |
| 25% Distribution | 25.2 nm |

### EXAMPLE 13

Tacrolimus (200 mg), Cholesteryl sulfate (120 mg), and Soy lecithin (7.88 g) were mixed together in water (70 mL) and homogenized using high pressure homogenizer. The resulting suspension was then filtered through 0.2 µ filter and then mixed with 7.5% sucrose (30 mL) and lyophilized both in vials and in bulk. The particle size was determined using Nicomp particle sizer 380. The mean volume weighting diameter amounted to less than 200 nm.

| **Mean/Distributions** | **Particle Size (Volume Weighting)** |
|---|---|
| Mean Volume Weighting Diameter | 76.4 nm |
| 99% Distribution | 240.8 nm |
| 90% Distribution | 134.3 nm |
| 80% Distribution | 105.1 nm |
| 75% Distribution | 95.8 nm |
| 50% Distribution | 65.9 nm |
| 25% Distribution | 45.5 nm |

The Tacrolimus lipid suspension was tested for toxicity in Balb/c mice. The single test dose at 10 mg/kg and 20 mg/kg was intravenously administered to mice. All the mice survived with no significant loss of body weight. Similarly, repeat dose toxicity study was conducted with a dose of 10 mg/kg or 20 mg/kg for consecutively 5 days with accumulated dose of 50 mg/kg and 100 mg/kg respectively. All the animals in the group survived. The results are reported in the table below as the number of mice surviving per total.

| **Treatment** | **Dose (mg/kg)** | **Survival/Total** |
|---|---|---|
| Single dose | 10 | 5/5 |
| | 20 | 5/5 |
| Repeat dose | 10 | 5/5 |
| | 20 | 5/5 |

### EXAMPLE 14

Cholesteryl sulfate (2.08 mg) and hydrogenated soyphosphatidylcholine (185.92 mg) in 0.9% aq. Sodium chloride solution (2 mL) was sonicated at 65° C for 30 minutes before Doxorubicin (40 mg) in 0.9% sodium chloride solution (2 mL) was added and further sonicated for 60 minutes. The formulation was lyophilized in the presence of 7.5% sucrose or and reconstituted in water for injection.

### EXAMPLE 15

Cholesteryl sulfate (20 mg) and soy lecithin (156.8 mg) in 0.9% aq. sodium chloride solution was sonicated at 65°C for 30 minutes before Doxorubicin (40 mg) in 0.9% sodium chloride solution (10 mL) was added and further sonicated for 60 minutes. The formulation is lyophilized in the presence of 7.5% sucrose and reconstituted in water for injection. The particle size was determined using Nicomp particle sizer 380. The mean volume diameter amounted to less than 200 nm.

### EXAMPLE 16

Docetaxel (20 mg), Cholesteryl sulfate (12.0 mg), and Soy lecithin (788 mg) were mixed together in water (10 mL) and using high pressure homogenizer. The formulation is lyophilized in the presence of 7.5% sucrose and reconstituted in water for injection. The particle size was determined using Nicomp particle sizer 380. The mean volume weighting diameter amounted to less than 200 nm.

| **Mean/Distributions** | **Particle Size (Volume Weighting)** |
|---|---|
| Mean Volume Weighting Diameter | 93.9 nm |
| 99% Distribution | 264.1 nm |
| 90% Distribution | 157.0 nm |
| 80% Distribution | 126.1 nm |
| 75% Distribution | 116.0 nm |
| 50% Distribution | 83.0 nm |
| 25% Distribution | 59.4 nm |

### EXAMPLE 17

Docetaxel (40 mg), Cholesteryl sulfate (24.0 mg), and Soy lecithin (1.57 g) were mixed together in water (10 mL) using high pressure homogenizer. The formulation is lyophilized in the presence of 7.5% sucrose and reconstituted in water for injection. The particle size was determined using Nicomp particle sizer 380. The mean volume diameter amounted to less than 200 nm

### EXAMPLE 18

Paclitaxel (20 mg), Cholesteryl sulfate (11.4 mg), and Soy lecithin (788.6 mg) were mixed together in water (10 mL) and homogenized using high pressure homogenizer. The formulation is lyophilized in the presence of 7.5% sucrose and reconstituted in water for injection. The particle size was determined using Nicomp particle sizer 380. The mean volume weighting diameter amounted to less than 200 nm

| **Mean/Distributions** | **Particle Size (Volume Weighting)** |
|---|---|
| Mean Volume Weighting Diameter | 124.1 nm |
| 99% Distribution | 357.4 nm |
| 90% Distribution | 209.6 nm |
| 80% Distribution | 167.4 nm |
| 75% Distribution | 153.7 nm |
| 50% Distribution | 108.9 nm |
| 25% Distribution | 77.2 nm |

The paclitaxel lipid suspension was tested for toxicity in Balb/c mice. The test dose (40 mg/kg) was intravenously administered to mice and the animals were monitored for 30 days. All the mice survived with no significant loss of body weight. Similarly, repeat dose toxicity study was conducted with a dose of 40 mg/kg for consecutively 5 days with accumulated dose of 200 mg/kg. All the animals in the group survived. The study was monitored for 30 days. The results are reported in the table below as the number of mice surviving per total

| **Treatment** | **Dose (mg/kg)** | **Survival/Total** |
|---|---|---|
| Single dose | 40 | 3/3 |
| Repeat dose | 40 | 4/4 |

### EXAMPLE 19

Paclitaxel (40 mg), Cholesteryl sulfate (22.8 mg), and Soy lecithin (1.58 g) were mixed together in water (10 mL) and homogenized using high pressure homogenizer. The formulation is lyophilized in the presence of 7.5% sucrose and reconstituted in water for injection. The particle size was determined using Nicomp particle sizer 380. The particle size data is shown in the table below.

| **Mean/Distributions** | **Particle Size (Volume Weighting)** |
|---|---|
| Mean Volume Weighting Diameter | 839.1 nm |
| 99% Distribution | 3425.8 nm |
| 90% Distribution | 1636.3 nm |
| 80% Distribution | 1185.7 nm |
| 75% Distribution | 1048.7 nm |
| 50% Distribution | 638.5 nm |
| 25% Distribution | 388.7 nm |

### REFERENCES

1. Abra, R. and Szoka, F.C.; PCT Appl WO8701933, 1987; Sanders, S. et al. 1991
2. Abra, R. US Patent 5,032,582, 1991**.**
3. Abra, R. US Patent 4,822,777, 1989
4. Abra, R. and Gua, L.S US Patent 5,194,266, 1993
5. Adler-Moore, J.; Jill, P.; Proffitt, R.t. J. Liposome Res. 1993, 2, 429-450.
6. Ahmad, I., Agarwal, A.; Pal, A.; Guru, P.Y.; Bachhawat, B.K. and Gupta, C.M. J. Biosciences, 1991, 14, 217-221.
7. Ahmad, I.; Sarkar, A.K.; and Bachhawat, B.K. Mol. Cell. Biochem. 1989, 91, 85-90.
8. Ahmad, I.; Sarkar, A.K.; and Bachhawat, B.K. Biot. Appl. Biochem. 1990, 12, 550-556.
9. Bissery, M-C.; Laborie, M.; Vacu, J.; Verrecchia, T. US Patent 6,146,663, 2000**.**
10. Brajtburg, J.; Powderly, W.G.; Kobayashi, G.S.; Medoff, G. Antimicrob. Agents and Chemother. 1990, 34, 381-384.
11. De Kruijff, B. and Demel, R.A.; Biochim. Biophys. Acta, 1974, 339, 57-70.
12. Deray G.; Mercadal, L.; Bagnis, C. Nephrologie, 2002, 23, 119-122.
13. Dumont, F.J. In:Libermann, R. Mukherjee, A. Eds. Drug development in transplantation and autoimmunity, Austin, TX: RG landes, 1996, 175.
14. Hammarstrom, L.; and Smith, C.I.E. Acta Patho. Microbial. Scand. 1977, 85, 277-283.
15. Hennenfent, K.L. and Govindan, R. Annals of Oncol. 2006, 17, 735-749.
16. Janoff, A.S.; Madden, T.D.; Cullis, P.R.; Kearns, J.J.; Durning, A.G.; U.S. Patent No. 6,406,713, 2002.
17. Ingle, G.R.; Sievers, T.M.; Holt, C.D. Ann Pharmacother, 2000, 34, 1044-1055.
18. Knoll, G.A. and Bell, R.C. BMJ, 1999, 318, 1104-1107.
19. Lister, J. Eur J. Haematol. 1996, 56(suppl 57), 18-23.
20. Little, J.R.; Plut E. J., Kotler-Brajtburg, J.; Mendoff, G. and Kobayashi, G.S. Immunochem., 1978, 15, 219-224.
21. Medoff, G. and Kobayashi, G.S. J. Am. Med. Assoc. 1975, 232, 619-620.
22. Podder, H.; Podbielski, J.; Hussein, I.; Katz, S.; van Buren, C.; Kahan, B.D.; Transpl. Int. 2001, 14, 135-142.
23. Proffitt, R.T.; Adler-Moore, J.; Fujii, G.; Satorius, A, Lee, M.J.A.; Bailey, A.; J. Controlled Release 1994, 28, 342-343.
24. Otsubo, T.; Maesaki, S.; Hossain, M.A.; Yamamoto, Y.; Tomono, K.; Tashiro, T.; Seki, J.; Tomii, Y.; Sonoke, S.; Kohno, S. J. Antimicrob. Agents and Chemother. 1999, 43, 471-475.
25. Proffitt, R.T.; Adler-Moore, J., Chiang, S-M US Patent 5,965,156 1999.
26. Proffitt, R.T.; Satorius, A.; Chiang, S. M.; Sullivan. L.; Adler-Moore, J.P. J. Antimicrob. Agents Chemother. 1991, 28(Suppl. B), 49-61.
27. Sanders, S.W.; Buchi, K.N.; Goddard, M.S.; Lang, J.K.; Tolman, K.G. Antimicrob. Agents and Chemother. 1991, 35, 1029-1034.
28. Ramos, H.; Brajtburg, J.; Marquez, V.; Cohen, B.E. Drugs Exptl. & Clin. Res. 1995, 21, 211-216.
29. Ramos, H.; Valdivieso, E.; Gamargo, M.; Dagger, F.; Cohen, B.E. J. Memo. Biol. 1996, 152, 6575.
30. Schiff, P.B.; Fant, J.; Horowitz, S.B. Nature, 1979, 227, 665-667.
31. Straubinger, R.M.; Sharma, A.; Mayhew, E. US Patent 5,415,869, 1995**.**
32. Szoka, F.C. Jr. US 5277914 A, 1994
33. Szoka, F.C. Jr,; Milholland, D.; Barza, M. Antimicrob. Agents and Chemother. 1987, 31, 421-429.
34. ten Tije, A.J.; Verweij, J.; Loos, W.J.; Sparreboom, A. Clin Pharmacokinet, 2003, 42, 665-685.
35. Valeriote, F.; Lynch, R.; Medoff, G.; and Kumar, B.V. J. Natl. Cancer. Inst. 1976, 56, 557-559.
36. Walsh, T.J.; Whitcomb, P.; Piscitelli, S. Figg, W.D.; Hill, S.; Chanock, S.J.; Jarosinski, P.; Gupta, R.; Pizzo, P.A. J. Antimicrob. Agents Chemother. 1997, 41, 1944-1948.

All references, including publications, patent applications, and patents cited herein, including those in the preceding list and otherwise cited in this specification, are hereby incorporated by reference to the same extent as if each reference was individually and specifically indicated to be incorporated by reference and were set forth in the entirely herein.

## Claims

1. An organic solvent-free process for preparing a lipid formulation of an active compound, wherein said process comprises:
a) preparing a suspension comprising at least one active compound and at least one lipid in a first aqueous medium at a pH between about pH 4.0 and pH 8.0, wherein said preparing of said suspension comprises a process consisting of:
i) providing:
1) a first aqueous medium consisting of water or water having buffer dissolved in it,
2) at least one partially water-soluble or water-insoluble active compound,
3) at least one phospholipid and at least one lipid selected from the group consisting of cholesterol or cholesterol sulfate and salts thereof, cholesterol hemisuccinate and salts thereof, cholesterol phosphate and salts thereof; and 4) one or more cryoprotectants;
ii) to said first aqueous medium, adding the at least one active compound and the at least one lipid; and
iii) mixing said first aqueous medium, said at least one active compound, and said at least one lipid to form a suspension;
b) treating said suspension to form a lipid-compound suspension, comprising lipid-compound complexes having a defined particle size;
c) lyophilizing said lipid-compound suspension comprising lipid-compound complexes having defined particle size in the presence of one or more cryoprotectants to form lyophilized material; and
d) reconstituting said lyophilized material with a second aqueous medium to obtain a lipid formulation of an active compound, said lipid formulation comprising a suspension of lipid-compound complexes of defined particle size, said defined particle size having a mean particle size of less than 5 microns.

2. The process of claim 1, wherein said second aqueous medium is same as or different from said first aqueous medium.

3. The process of any of claims 1-2, wherein said at least one active compound comprises a compound selected from the group consisting of docetaxel, paclitaxel, doxorubicin, epirubicin, tamoxifen, endoxifen, etoposide, anthracyclines , amphotericin B, tacrolimus, and sacrolimus, cyclosporine, and methotrexate.

4. The process of any of claims 1-3, wherein said lipid preparation further comprises polyethylene glycol.

5. The process of any of claims 1-4, wherein said at least one lipid comprises a cholesterol or cholesterol derivative, wherein the mole ratio of active compound to cholesterol or cholesterol derivative is between about 10:1 and 1:10.

6. The process of any of claims 1-5, wherein said at least one phospholipid comprises hydrogenated soy phosphatidylcholine or soy phosphatidylcholine, wherein the mole ratio of active compound and hydrogenated soy phosphatidylcholine or soy phosphatidylcholine is between about 1:1 to about 1:100.

7. The process of any of claims 1-6, wherein said lipid preparation comprises a total lipid concentration of from 2.5% by weight to about 95% by weight.

8. The process of any of claims 1-7, wherein the weight to weight ratio of active compound to lipid in said lipid preparation is between 1:10 to 1:100,

9. The process of any of claim 1-8, wherein said treating said suspension comprises extruding said suspension through a selected size aperture and/or treating by high pressure split homogenization.

## Patentansprüche

1. Verfahren ohne organische Lösungsmittel zur Herstellung einer Lipid-Formulierung einer Wirkverbindung, wobei das Verfahren das Folgende umfasst:
a) Herstellen einer Suspension, die wenigstens eine Wirkverbindung und wenigstens ein Lipid umfasst, in einem ersten wässrigen Medium bei einem pH zwischen ungefähr pH 4,0 und pH 8,0, wobei die Herstellung der Suspension ein Verfahren umfasst, das aus Folgendem besteht:
i) Bereitstellung:
1) eines ersten wässrigen Mediums, das aus Wasser oder Wasser mit darin gelöstem Puffer besteht,
2) wenigstens einer teilweise wasserlöslichen oder wasserunlöslichen Wirkverbindung,
3) wenigstens eines Phospholipids und wenigstens eines Lipids, das aus der Gruppe ausgewählt ist, die aus Cholesterin oder Cholesterinsulfat und Salzen davon, Cholesterinhemisuccinat und Salzen davon, Cholesterinphosphat und Salzen davon besteht; und
4) eines oder mehrerer Kälteschutzmittel;
ii) Hinzufügen der wenigstens einen Wirkverbindung und des wenigstens einen Lipids zu dem ersten wässrigen Medium; und
iii) Mischen des ersten wässrigen Mediums, der wenigstens einen Wirkverbindung und des wenigstens einen Lipids zur Bildung einer Suspension;
b) Behandlung der Suspension zur Bildung einer Lipid-Verbindungs-Suspension, die Lipid-Verbindungs-Komplexe mit einer definierten Partikelgröße umfasst;
c) Lyophilisation der Lipid-Verbindungs-Suspension, die Lipid-Verbindungs-Komplexe mit einer definierten Partikelgröße umfasst, in Anwesenheit von einem oder mehreren Kälteschutzmitteln zur Bildung von lyophilisiertem Material; und
d) Rekonstitution des lyophilisierten Materials mit einem zweiten wässrigen Medium, um eine Lipid-Formulierung einer Wirkverbindung zu erhalten, wobei die Lipid-Formulierung eine Suspension von Lipid-Verbindungs-Komplexen mit einer definierten Partikelgröße umfasst, wobei die definierte Partikelgröße eine mittlere Partikelgröße von unter 5 Mikron aufweist.

2. Verfahren nach Anspruch 1, wobei das zweite wässrige Medium dem ersten wässrigen Medium gleicht oder davon verschieden ist.

3. Verfahren nach einem der Ansprüche 1-2, wobei die wenigstens eine Wirkverbindung eine Verbindung umfasst, die aus der aus Docetaxel, Paclitaxel, Doxorubicin, Epirubicin, Tamoxifen, Endoxifen, Etoposid, Anthracyclinen, Amphotericin B, Tacrolimus, und Sacrolimus, Cyclosporin, und Methotrexat bestehenden Gruppe ausgewählt ist.

4. Verfahren nach einem der Ansprüche 1-3, wobei die Lipidzubereitung ferner Polyethylenglykol umfasst.

5. Verfahren nach einem der Ansprüche 1-4, wobei das wenigstens eine Lipid ein Cholesterin oder Cholesterin-Derivat umfasst, wobei das Molverhältnis von Wirkverbindung zur Cholesterin oder Cholesterin-Derivat zwischen ungefähr 10:1 und 1:10 beträgt.

6. Verfahren nach einem der Ansprüche 1-5, wobei das wenigstens eine Phospholipid hydriertes Soja-Phosphatidylcholin oder Soja-Phosphatidylcholin umfasst, wobei das Molverhältnis von Wirkverbindung und hydriertem Soja-Phosphatidylcholin oder Soja-Phosphatidylcholin zwischen ungefähr 1:1 und ungefähr 1:100 beträgt.

7. Verfahren nach einem der Ansprüche 1-6, wobei die Lipidzubereitung eine Gesamtlipidkonzentration von 2,5 Gewichts-% bis ungefähr 95 Gewichts-% umfasst.

8. Verfahren nach einem der Ansprüche 1-7, wobei das Gewichts-Gewichts-Verhältnis von Wirkverbindung zu Lipid in der Lipidzubereitung zwischen 1:10 zu 1:100 beträgt.

9. Verfahren nach einem der Ansprüche 1-8, wobei die Behandlung der Suspension die Extrusion der Suspension durch eine Öffnung ausgewählter Größe und/oder Behandlung durch Hochdruckspalthomogenisation umfasst.

## Revendications

1. Procédé sans solvant organique pour la préparation d'une formulation lipidique d'un composé actif, dans lequel ledit procédé comprend :
a) la préparation d'une suspension comprenant au moins un composé actif et au moins un lipide dans un premier milieu aqueux à un pH situé entre environ le pH 4,0 et le pH 8,0, dans lequel ladite préparation de ladite suspension comprend un procédé consistant à :
i) apporter :
1) un premier milieu aqueux constitué d'eau ou d'eau dans laquelle un tampon est dissous,
2) au moins un composé actif partiellement soluble dans l'eau ou insoluble dans l'eau,
3) au moins un phospholipide et au moins un lipide choisis dans le groupe constitué du cholestérol ou du sulfate de cholestérol et des sels de celui-ci, de l'hémisuccinate de cholestérol et des sels de celui-ci, du phosphate de cholestérol et des sels de celui-ci ; et
4) un ou plusieurs cryoprotecteurs ;
ii) audit premier milieu aqueux, ajouter l'au moins un composé actif et l'au moins un lipide ; et
iii) mélanger ledit premier milieu aqueux, ledit au moins un composé actif, et ledit au moins un lipide pour former une suspension ;
b) traiter ladite suspension pour former une suspension de composé lipidique, comprenant des complexes lipide-composé ayant une taille de particule définie ;
c) lyophiliser ladite suspension de composé lipidique comprenant des complexes lipide-composé ayant une taille de particule définie en présence d'un ou plusieurs cryoprotecteurs pour former un matériau lyophilisé ; et
d) reconstituer ledit matériau lyophilisé à l'aide d'un second milieu aqueux pour obtenir une formulation lipidique d'un composé actif, ladite formulation lipidique comprenant une suspension de complexes lipide-composé ayant une taille de particule définie, ladite taille de particule définie ayant une taille moyenne de particule inférieure à 5 microns.

2. Procédé selon la revendication 1, dans lequel ledit second milieu aqueux est identique audit premier milieu aqueux ou différent dudit premier milieu aqueux.

3. Procédé selon l'une quelconque des revendications 1 à 2, dans lequel ledit au moins un composé actif comprend un composé choisi dans le groupe constitué du docétaxel, du paclitaxel, de la doxorubicine, de l'épirubicine, du tamoxifène, de l'endoxifène, de l'étoposide, des anthracyclines, de l'amphotéricine B, du tacrolimus et du sacrolimus, de la cyclosporine et du méthotrexate.

4. Procédé selon l'une quelconque des revendications 1 à 3, dans lequel ladite préparation lipidique comprend en outre du polyéthylène glycol.

5. Procédé selon l'une quelconque des revendications 1 à 4, dans lequel ledit au moins un lipide comprend un cholestérol ou un dérivé du cholestérol, dans lequel le rapport molaire du composé actif au cholestérol ou au dérivé du cholestérol se situe entre environ 10/1 et 1/10.

6. Procédé selon l'une quelconque des revendications 1 à 5, dans lequel ledit au moins un phospholipide comprend de la phosphatidylcholine de soja hydrogénée ou de la phosphatidylcholine de soja, dans lequel le rapport molaire du composé actif et de la phosphatidylcholine de soja hydrogénée ou de la phosphatidylcholine de soja se situe entre environ 1/1 et environ 1/100.

7. Procédé selon l'une quelconque des revendications 1 à 6, dans lequel ladite préparation lipidique comprend une concentration totale de lipides de 2,5 % en poids à environ 95 % en poids.

8. Procédé selon l'une quelconque des revendications 1 à 7, dans lequel le rapport du poids au poids du composé actif aux lipides dans ladite préparation lipidique se situe entre 1/10 et 1/100,

9. Procédé selon l'une quelconque des revendications 1 à 8, dans lequel ledit traitement de ladite suspension comprend l'extrusion de ladite suspension à travers une ouverture de taille choisie et/ou le traitement par une homogénéisation par séparation à haute pression.
